(19) Europäisches Patentamt
European Patent Office
Office européen des brevets

(11) **EP 3 558 671 B1**

(12) **EUROPÄISCHE PATENTSCHRIFT**

(45) Veröffentlichungstag und Bekanntmachung des Hinweises auf die Patenterteilung:
**30.08.2023  Patentblatt 2023/35**

(21) Anmeldenummer: **17821655.2**

(22) Anmeldetag: **23.12.2017**

(51) Internationale Patentklassifikation (IPC):
**B32B 18/00** (2006.01)    **C04B 35/486** (2006.01)
**C04B 35/638** (2006.01)

(52) Gemeinsame Patentklassifikation (CPC):
**B32B 18/00; C04B 35/486; C04B 35/638;**
C04B 2235/3206; C04B 2235/3217;
C04B 2235/3224; C04B 2235/3225;
C04B 2235/3227; C04B 2235/3241;
C04B 2235/3265; C04B 2235/3272;
C04B 2235/616; C04B 2235/6562;
C04B 2235/6567; C04B 2235/661;      (Forts.)

(86) Internationale Anmeldenummer:
**PCT/EP2017/084571**

(87) Internationale Veröffentlichungsnummer:
**WO 2018/115529 (28.06.2018 Gazette 2018/26)**

(54) **DIE VERWENDUNG EINES MEHRSCHICHTIGEN OXIDKERAMIKKÖRPERS MIT ANGEPASSTEM SINTERVERHALTEN**

THE USE OF MULTILAYERED OXIDE CERAMIC BODIES WITH ADAPTED SINTERING BEHAVIOUR

L'UTILISATION DE CORPS MULTICOUCHE EN CÉRAMIQUE OXYDÉE PRÉSENTANT UN COMPORTEMENT AU FRITTAGE ADAPTÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2016  EP 16206751**

(43) Veröffentlichungstag der Anmeldung:
**30.10.2019  Patentblatt 2019/44**

(73) Patentinhaber: **Ivoclar Vivadent AG**
**9494 Schaan (LI)**

(72) Erfinder:
• **REINSHAGEN, Jörg**
**75177 Pforzheim (DE)**
• **MEYHÖFER, Henning**
**70197 Stuttgart (DE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**EP-A1- 3 045 435      DE-A1- 1 538 759**
**US-A1- 2009 226 790   US-A1- 2010 001 828**
**US-A1- 2015 282 905**

• **MIURA M ET AL: "FORMATION OF PLATE-LIKE LANTHANUM-B-ALUMINATE CRYSTAL IN CE-TZP MATRIX", JOURNAL OF MATERIALS SCIENCE, KLUWER ACADEMIC PUBLISHERS, DORDRECHT, Bd. 29, Nr. 1, 1. Januar 1994 (1994-01-01) , Seiten 262-268, XP000433025, ISSN: 0022-2461, DOI: 10.1007/BF00356602**

- **HAIYAN CHEN ET AL: "Dynamic characteristics of functionally gradient piezoelectric actuators", PROPERTIES AND APPLICATIONS OF DIELECTRIC MATERIALS, 2000. PROCEEDINGS OF THE 6TH INTERNATIONAL CONFERENCE ON JUNE 21-26, 2000, PISCATAWAY, NJ, USA,IEEE, Bd. 2, 21. Juni 2000 (2000-06-21), Seiten 983-987, XP010516251, ISBN: 978-0-7803-5459-3**
- **JIN D ET AL: "Functionally Graded Piezoelectric Ceramics for Ultrasonic Transducers", KEY ENGINEERING MATERIALS, TRANS TECH PUBLICATIONS LTD., STAFA-ZURICH, CH, Bd. 336-338, Nr. 3, 1. Januar 2007 (2007-01-01), Seiten 2609-2612, XP009503674, ISSN: 1013-9826, DOI: 10.4028/WWW.SCIENTIFIC.NET/KEM.336-338.26 0 9**

(52) Gemeinsame Patentklassifikation (CPC): (Forts.)
C04B 2235/72; C04B 2235/75; C04B 2235/96;
C04B 2235/9638; C04B 2235/9661;
C04B 2237/068; C04B 2237/348; C04B 2237/582

## Beschreibung

Die Erfindung betrifft die Verwendung mehrschichtiger Oxidkeramikkörper und insbesondere vorgesinterter mehrschichtiger Oxidkeramikrohlinge und Oxidkeramik-Grünkörper zur Herstellung von Dentalrestaurationen. Diese Körper können durch weiteres Sintern verzugsfrei thermisch verdichtet werden und sind daher besonders geeignet zur Herstellung von Dentalrestaurationen mit hervorragenden mechanischen Eigenschaften und sehr hoher Passgenauigkeit und erlauben es, die optischen Eigenschaften natürlicher Zähne sehr genau zu imitieren. Die Erfindung betrifft auch ein Verfahren zur Herstellung von solchen mehrschichtigen Oxidkeramikkörpern sowie ein Verfahren zur Herstellung von Dentalrestaurationen unter Verwendung der mehrschichtigen Oxidkeramikkörper.

Oxidkeramikmaterialien werden seit vielen Jahren zur Herstellung von dentalen Implantaten und Restaurationen verwendet. Solche Keramiken basieren üblicherweise auf Zirkonoxid in Form von tetragonalem Zirkonoxid-Polykristall (TZP). Reines $ZrO_2$ unterliegt bei Temperaturen unterhalb von 950°C einer Phasenumwandlung von tetragonal zu monoklin, die mit einer erheblichen Volumenzunahme einhergeht. Zirkonoxid bei Raumtemperatur in seiner tetragonalen Form zu halten, erfordert die Verwendung von Additiven wie $Y_2O_3$, $CeO_2$, MgO oder CaO. Diese Additive inhibieren die Umwandlung von tetragonal zu monoklin, was zu einem metastabilen Zustand führt, in dem Zirkonoxid ganz oder teilweise in seiner tetragonalen Form vorliegt. Wenn sich in solchem metastabilen tetragonalem Zirkonoxid-Keramiken Risse bilden, löst die Spannung an der Rissspitze eine lokale Umwandlung von der tetragonalen in die monokline Form aus, und die damit verbundene Volumenzunahme wirkt einer Rissfortpflanzung wirksam entgegen. Dieser sogenannte Umwandlungsverstärkungsmechanismus sorgt für eine hohe Festigkeit von stabilisierten Zirkonoxid-Keramiken (Hannink et al., J. Am. Ceram. Soc. 2000, 83, 461-487). Zusammen mit den bioinerten Eigenschaften von Zirkonoxid hat dies zur Verwendung von dotiertem TZP in der Orthopädie und für Dentalrestaurationen geführt. Heute werden insbesondere mit $Y_2O_3$ stabilisierter tetragonaler Zirkonoxid-Polykristall (Y-TZP), üblicherweise mit Zusatz von $Al_2O_3$ (etwa 0,25 Gew.-%), weithin als vollkeramisches dentales Biomaterial verwendet (Denry et al., Dental Materials 2008, 24, 299-307).

Zur Herstellung von Keramiken sind verschiedene Verfahren bekannt. Bevorzugte Verfahren sind (i) axiales Pressen oder kaltisostatisches Pressen (CIP) gefolgt von konventionellem Sintern, (ii) Schlickergießen gefolgt von konventionellem Sintern und (iii) Heißpressen (HP) oder heißisostatisches Pressen (HIP).

Für dentale Anwendungen umfasst die Herstellung von Keramikmaterialien oft zwei Verdichtungsschritte, die durch einen Formgebungsschritt getrennt sind. Folglich können die Keramikmaterialien gepresst oder gegossen und dann zu einem intermediären offenporigen Zustand vorgesintert werden. Dann kann eine Formgebung oder Vorformgebung des Keramikmaterials durchgeführt werden, an die sich eine abschließende thermische Verdichtung durch weiteres Sintern anschließt.

Um die ästhetische Erscheinung von Dentalrestaurationen zu verbessern, müssen Keramiken zur Verwendung als dentale Restaurationsmaterialien häufig mit einer Einfärbung versehen werden. Zur Einfärbung von Keramikmaterialien sind eine Reihe von Möglichkeiten bekannt.

Ein Ansatz zum Erhalt von gefärbten Dentalkeramikmaterialien verwendet die Einfärbung eines Keramikmaterials im porösen Zustand durch Infiltration von Lösungen von Metallverbindungen. Üblicherweise wird dabei die poröse Struktur getrocknet, ganz oder teilweise mit einer färbenden Lösung infiltriert, die Oberfläche gereinigt und die infiltrierte Struktur getrocknet, gegebenenfalls die poröse Struktur mit einer weiteren färbenden Lösung infiltriert und schließlich gesintert.

US 6,709,694 B1 beschreibt ein Verfahren zur Einfärbung von Oxidkeramiken im porösen oder absorbierenden Zustand mit Lösungen von Salzen oder Komplexen von Übergangsmetallen.

EP 1 486 476 A1 beschreibt ein Verfahren zum Einfärben von vorgesinterten Keramikkörpern unter Verwendung einer Lösung, die ein Metallsalz, ein Lösungsmittel und ein Polyethylenglycol mit einem $M_n$ im Bereich von 1.000 bis 200.000 enthält.

Dieser Ansatz leidet unter dem für den Dentaltechniker komplizierten Verfahren und der geringen Homogenität der erhaltenen Farbverteilung. Außerdem kann sich in Abhängigkeit von der Konzentration der färbenden Ionen der Vergrößerungsfaktor innerhalb der gefärbten Schichten ändern, was während des abschließenden Sinterschritts zu Spannungen zwischen den gefärbten und nichtgefärbten Bereichen führt.

Ein anderer Ansatz beinhaltet die Voreinfärbung eines Zirkonoxidpulvers durch Kopräzipitation von Zirkonoxid zusammen mit färbenden Verbindungen oder durch Kontaktieren eines Zirkonoxidpulvers mit Lösungen von färbenden Verbindungen um voreingefärbte Primär- und Sekundärpartikel mit verschiedenen Pulvereigenschaften zu erhalten.

US 5,011,403 A beschreibt die Herstellung einer gefärbten Klammer durch Verpressen und Sintern eines Pulvers, das durch Zugabe von färbenden Übergangsmetalloxiden zu einem teilstabilisierten Zirkonoxidpulver erhalten wurde, wobei die Übergangsmetalloxide entweder in Pulverform oder durch Atomisieren des Zirkonoxidpulvers mit einer Lösung von wasserlöslichen Salzen des Übergangsmetalloxids eingebracht werden.

US 5,263,858 A beschreibt die Herstellung eines elfenbeinfarbenen gesinterten Zirkonoxidkörpers, der als Klammer für orthodontische Anwendungen verwendet werden kann, wobei ein gemischtes Pulver durch (A) Kopräzipitation einer Lösung, die Verbindungen von Zirkonium, eines Stabilisators, Erbium und Praseodym enthält, und Kalzinierung oder (B) Mischen von Lösungen von Verbindungen von Erbium und Praseodym mit einem einen Stabilisator enthaltenden Zirkonoxidpulver hergestellt wird und aus dem resultierenden Pulver ein geformter Körpers gebildet und gesintert wird.

US 5,656,564 A beschreibt gefärbte gesinterte Zirkonoxidkörper für orthodontische Klammermaterialien, die durch Feuchtvermischung eines stabilisierten Zirkonoxidpulvers mit färbenden Substanzen, Formgebung des erhaltenen Pulvers und Sintern hergestellt werden.

US 6,713,421 A beschreibt Rohlinge auf Basis einer Keramikzusammensetzung, die Zirkonoxid, mindestens eines der Oxide von Aluminium, Gallium, Germanium und Indium und außerdem färbende Additive enthält. Die Keramikzusammensetzung wird durch Kopräzipitation und Kalzinierung hergestellt.

Ein weiterer Ansatz beinhaltet die Einfärbung von direktverpressbaren Keramikpulvern durch Beschichtungstechniken.

US 2007/292597 A1 beschreibt ein Verfahren zur Herstellung von ein- und mehrfarbigen Rohlingen und dentalen Formteilen, bei dem ein Oxidpulver mit einer färbenden Substanz beschichtet wird, das eingefärbte Pulver verpresst wird, um einen Formkörper zu bilden, und der verpresste Formkörper gesintert wird.

US 2008/0274440 A1 beschreibt einen Stiftaufbau für ein dentales Implantat, der eine aus einem Stück bestehende Stützstruktur für eine dentale Prothese umfasst, die aus einem Keramikmaterial hergestellt und eingefärbt wird, um die Farbe der dentalen Prothese an das umgebende Zahnmaterial und Zahnfleischgewebe anzupassen. Die Einfärbung des Stiftaufbaus kann unter anderem durch Beschichten eines Oxidpulvers mit färbenden Substanzen gemäß US 2007/292597 A1 erreicht werden.

Noch ein anderer Ansatz beinhaltet das Einfärben durch Abmischen von gefärbten und nichtgefärbten Pulvern und Pigmenten.

US 6,379,593 beschreibt ein Verfahren zur Herstellung eines mehrfarbigen Formkörpers, der zur Weiterverarbeitung unter Bildung einer Dentalrestauration geeignet ist, bei dem nacheinander unterschiedlich gefärbte Keramikmaterialien in eine Pressdüse eingebracht, zur Form eines Formkörpers verpresst und gesintert werden.

US 2007/272120 A1 beschreibt einen Keramikblock, der erste und zweite keramische Verbindungen mit unterschiedlichen optischen Eigenschaften und ferner einen Übergangsbereich zwischen den keramischen Verbindungen aufweist, in dem der Gradient der Änderung der resultierenden optischen Eigenschaften im Wesentlichen konstant ist.

US 2008/064011 A1 beschreibt einen mehrfarbigen Formkörper mit unterschiedlich gefärbten Hauptschichten und Zwischenschichten, bei dem eine Farbänderung zwischen den Zwischenschichten in einer Richtung erfolgen, die der Richtung der Farbeänderung zwischen den Hauptschichten entgegengesetzt ist. Ebenfalls offenbart ist ein mehrfarbiger Formkörper mit unterschiedlich gefärbten Hauptschichten und einer Zwischenschicht, die eine Mischung der Materialien der Hauptschichten enthält.

WO 2008/083358 A1 beschreibt einen mehrfarbigen Dentalrohling mit konzentrischen inneren und äußeren Zonen mit unterschiedlicher Farbgebung.

US 2010/0216095 A1 beschreibt die Herstellung von eingefärbten Dentalkeramiken, indem Y-TZP mit färbenden Komponenten gemischt wird, um im Wesentlichen homogene Aggregatmaterialien zu erhalten, unterschiedlich gefärbte Aggregatmaterialien gemischt werden und gepresst und gesintert wird.

US 2011/0189636 A1 beschreibt geformte Körper, die unterschiedlich gefärbte erste und zweite Komponenten enthalten, wobei die zweite Komponente innerhalb der ersten Komponente unter Bildung einer gekrümmten Grenzfläche angeordnet ist.

US 2012/139141 A1 beschreibt die Herstellung von gefärbten Zirkonoxidprodukten, indem ein Y-TZP-Pulver mit einer Lösung von Färbemitteln behandelt wird, um ein pigmentierten Pulver zu erhalten, das pigmentierte Pulver mit einem ungefärbten Pulver gemischt wird und das gemischte Pulver verpresst und gesintert wird.

Es hat sich gezeigt, dass die Verfahren nach dem Stand der Technik an dem Problem von unterschiedlichen und inkompatiblen Sinterkinetiken der darin eingesetzten unterschiedlichen Keramikpulver, wie Kombinationen von gefärbten und nichtgefärbten Pulvern oder Kombinationen von unterschiedlich gefärbten Pulvern, leiden. Wenn unterschiedliche Pulver kombiniert werden, um die unterschiedlichen Schichten eines mehrschichtigen Keramikkörpers zu bilden, führen diese Unterschiede der Sinterkinetik zu einem Verzug des Körpers beim Sintern. Ein solcher Verzug ist besonders im Falle von dentalen Anwendungen ungeeignet.

WO 2015/011079 A1 beschreibt mehrschichtige Oxidkeramikrohlinge zur Herstellung von Dentalrestaurationen und Verfahren zu deren Herstellung, bei denen ein Sinterverhalten der verschiedenen Schichten angepasst wird. Es hat sich jedoch gezeigt, dass die Farb- und insbesondere die Transluzenzeigenschaften der Oxidkeramiken zur Nachahmung der Eigenschaften natürlicher Zähne nicht in allen Fällen optimal sind.

US2015/282905 zeigt einen mehrschichtigen Oxidkeramik-Grünkörper aus den Teilen A und B. Die beiden Teile A und B enthalten Zirkonoxid, dotiert mit sowohl Lanthan- als auch Yttriumoxid, aber A und B haben sowohl für das Lanthan- als auch für das Yttriumoxid unterschiedliche Mengen. Die Grünkörper werden bei 900°C vorgesintert und danach bei 1250°C weiter gesintert. Teil A ist 75% tetragonal und 25% kubisch, teil B 25% tetragonal und 75% kubisch.

Teil A enthält 3,75 Gew.-% La 2O3. Teil B enthält 1,25 Gew.-% La2O3. Das Produkt wird als poröse Dentalkeramik verwendet.

Der Erfindung liegt daher die Aufgabe zugrunde, mehrschichtige Oxidkeramikkörper und insbesondere Oxidkeramikrohlinge mit unterschiedlich gefärbten Schichten bereitzustellen, die während der thermischen Verdichtung keinen Verzug zeigen, und insbesondere Keramikkörper bereitzustellen, die zur Herstellung von Dentalprodukten mit hoher Passgenauigkeit, einer verlässlichen und einfachen Bearbeitung durch den Dentaltechniker und einer sehr guten ästhetischen Erscheinung der abschließend verdichteten Keramik geeignet sind.

Diese Aufgabe wird durch die Verwendung eines vorgesinterten mehrschichtigen Oxidkeramikrohlings nach den Ansprüchen 1 bis 18, 21 und 26 gelöst. Gegenstand der Erfindung ist auch die Verwendung eines mehrschichtigen Oxidkeramik-Grünkörpers nach den Ansprüchen 19 bis 21 und 26, das Verfahren zur Herstellung eines Rohlings oder Grünkörpers nach den Ansprüchen 22 bis 25 sowie das Verfahren zur Herstellung einer Dentalrestauration nach den Ansprüchen 27 bis 30.

Der erfindungsgemäß verwendete vorgesinterte mehrschichtige Oxidkeramikrohling zeichnet sich dadurch aus, dass er mindestens zwei unterschiedliche Schichten umfasst, wobei die Oxidkeramik auf Zirkonoxid basiert und wobei mindestens eine Schicht 0,005 bis 1,0 Gew.-% $La_2O_3$ enthält und sich die mindestens zwei unterschiedlichen Schichten in ihrem Gehalt an $La_2O_3$ unterscheiden.

Es hat sich überraschenderweise gezeigt, dass die erfindungsgemäß verwendeten Rohlinge keinen oder im wesentlichen keinen Verzug während des Vorsinterns und während des abschließenden Sinterns zeigen. Insbesondere leiden sie nicht unter signifikanten Unterschieden in der Sinterkinetik oder im Schrumpfungsverhalten zwischen den unterschiedlichen Schichten. Dabei hat sich überraschend herausgestellt, dass die Zugabe von $La_2O_3$ zur Anpassung des Sinterverhaltens der unterschiedlichen Schichten in besonderer Weise geeignet ist. Insbesondere ermöglicht die Zugabe von $La_2O_3$ den Ausgleich von unterschiedlichem Sinterverhalten, das durch unterschiedliche Eigenschaften der Ausgangspulver wie spezifische BET-Pulveroberfläche, Partikelgröße, Partikelform, Pressverhalten und chemische Zusammensetzung, insbesondere durch Additive zur Verbesserung der Farb- und Transluzenzeigenschaften, hervorgerufen wird.

Erfindungsgemäß sind solche Rohlinge bevorzugt, bei denen mindestens eine Schicht 0,01 bis 1,0 Gew.-%, bevorzugt 0,025 bis 0,5 Gew.-% und am meisten bevorzugt 0,03 bis 0,20 Gew.-% $La_2O_3$ enthält.

Weiter bevorzugt sind Rohlinge, bei denen ferner mindestens eine Schicht $Al_2O_3$ und/oder $MgO$ enthält. Es hat sich gezeigt, dass das Sinterverhaltens der unterschiedlichen Schichten durch die zusätzliche Verwendung von $Al_2O_3$ und $MgO$ besonders gut angepasst werden kann.

Dabei sind solche Rohlinge bevorzugt, bei denen die mindestens eine Schicht 0,001 bis 5 Gew.-%, insbesondere 0,005 bis 1,0 Gew.-%, bevorzugt 0,01 bis 0,20 Gew.-% und am meisten bevorzugt 0,02 bis 0,10 Gew.-% $Al_2O_3$ und/oder $MgO$ enthält.

Insbesondere sind solche Rohlinge bevorzugt, bei denen die mindestens eine Schicht 0,001 bis 5 Gew.-%, insbesondere 0,005 bis 1,0 Gew.-%, bevorzugt 0,01 bis 0,10 Gew.-% und am meisten bevorzugt 0,02 bis 0,05 Gew.-% $Al_2O_3$ enthält. Ebenso sind solche Rohlinge bevorzugt, bei denen die mindestens eine Schicht 0,001 bis 5 Gew.-%, insbesondere 0,005 bis 1,0 Gew.-%, bevorzugt 0,01 bis 0,10 Gew.-% und am meisten bevorzugt 0,01 bis 0,03 Gew.-% $MgO$ enthält.

Weiter bevorzugt sind Rohlinge, bei denen die mindestens eine Schicht $Al_2O_3$ und $MgO$ in einem Gewichtsverhältnis von 10:1 bis 1:10, vorzugsweise 5:1 bis 1:1 und besonders bevorzugt 4:1 bis 2:1 enthält.

Ganz besonders bevorzugt sind solche Rohlinge, bei denen mindestens eine Schicht $La_2O_3$ in den oben definierten Mengen enthält und mindestens eine andere Schicht $Al_2O_3$ und/oder $MgO$, insbesondere in den oben definierten Mengen, enthält.

In einer bevorzugten Ausführungsform enthält mindestens eine Schicht und vorzugsweise enthalten alle Schichten des erfindungsgemäß verwendeten Rohlings $Y_2O_3$. Dabei ist es bevorzugt, dass mindestens eine und vorzugsweise alle Schichten 0,1 bis 20,0 Gew.-%, insbesondere 1,0 bis 15,0 Gew.-%, bevorzugt 5,0 bis 12,5 Gew.-% und am meisten bevorzugt 7,0 bis 9,5 Gew.-% $Y_2O_3$ enthalten. Es hat sich gezeigt, dass die Zugabe von $Y_2O_3$ besonders geeignet ist, um den Rohlingen und den daraus hergestellten Dentalprodukten Farb- und insbesondere Transluzenzeigenschaften zu verleihen, die die entsprechenden Eigenschaften des natürlichen Zahnmaterials besonders gut imitieren können. Weiter ist es bevorzugt, dass sich die mindestens zwei unterschiedlichen Schichten in ihrem Gehalt an $Y_2O_3$ unterscheiden. Dabei ist es besonders bevorzugt, dass die Differenz im $Y_2O_3$-Gehalt zwischen der Schicht mit dem niedrigsten $Y_2O_3$-Gehalt und der Schicht mit dem höchsten $Y_2O_3$-Gehalt mindestens 1,0 Gew.-%, vorzugsweise mindestens 1,5 Gew.-% und insbesondere mindestens 1,8 Gew.-%, weiter bevorzugt mindestens 2,0 Gew.-% und insbesondere mindestens 2,5 Gew.-%, noch weiter bevorzugt mindestens 3,0 Gew.-% und insbesondere mindestens 3,5 Gew.-% und am meisten bevorzugt mindestens 3,8 Gew.-% beträgt.

Dabei ist es erfindungsgemäß besonders bevorzugt, dass die Schicht mit dem niedrigsten $Y_2O_3$-Gehalt $La_2O_3$ enthält und/oder die Schicht mit dem höchsten $Y_2O_3$-Gehalt $Al_2O_3$ und/oder $MgO$ enthält. Es hat sich überraschend gezeigt, dass die Verwendung von $La_2O_3$ in Schichten mit niedrigem $Y_2O_3$-Gehalt einerseits und die Verwendung von

$Al_2O_3$ und/oder MgO in Schichten mit hohem $Y_2O_3$-Gehalt andererseits zur Anpassung des Sinterverhaltens von Schichten mit unterschiedlichen Gehalten an $Y_2O_3$ besonders geeignet ist.

Besonders bevorzugt enthält die Schicht mit dem niedrigsten $Y_2O_3$-Gehalt 0,005 bis 1,0 Gew.-%, insbesondere 0,01 bis 1,0 Gew.-%, bevorzugt 0,25 bis 0,9 Gew.-% und am meisten bevorzugt 0,5 bis 0,8 Gew.-% $La_2O_3$. Weiter bevorzugt enthält die Schicht mit dem höchsten $Y_2O_3$-Gehalt 0,001 bis 5 Gew.-%, insbesondere 0,005 bis 1,0 Gew.-%, bevorzugt 0,01 bis 0,20 Gew.-% und am meisten bevorzugt 0,02 bis 0,10 Gew.-% $Al_2O_3$ und/oder MgO.

In einer besonders bevorzugten Ausführungsform des erfindungsgemäß verwendeten Rohlings berechnet sich in jeder der mindestens zwei unterschiedlichen Schichten der $La_2O_3$-Gewichtsanteil nach der folgenden Formel:

$$m(La_2O_3) = m_{min}(La_2O_3) + (m_{max}(Y_2O_3) - m(Y_2O_3)) * f,$$

wobei

| | |
|---|---|
| $m(La_2O_3)$ | der $La_2O_3$-Gewichtsanteil in der jeweiligen Schicht ist, |
| $m_{min}(La_2O_3)$ | der minimale $La_2O_3$-Gewichtsanteil aller Schichten ist, |
| $m(Y_2O_3)$ | der $Y_2O_3$-Gewichtsanteil in der jeweiligen Schicht ist, |
| $m_{max}(Y_2O_3)$ | der maximale $Y_2O_3$-Gewichtsanteil aller Schichten ist, und |
| $f$ | im Bereich von 0,01 bis 1,00, insbesondere im Bereich von 0,03 bis 0,20, bevorzugt im Bereich von 0,06 bis 0,10, besonders bevorzugt im Bereich von 0,065 bis 0,085 und am meisten bevorzugt im Bereich von 0,081 bis 0,083 liegt. |

Oxidkeramiken sind allgemein hochkristalline Keramikmaterialien, die auf Oxidverbindungen basieren und allenfalls einen sehr geringen Anteil an Glasphase aufweisen. Typische Oxidkeramiken basieren auf $ZrO_2$, $Al_2O_3$, $TiO_2$, MgO, Kombinationen, Mischkristallen und Kompositen davon. Erfindungsgemäß basiert die Oxidkeramik auf Zirkonoxid. Oxidkeramiken auf Basis von $ZrO_2$ und $Al_2O_3$ sind besonders bevorzugt.

Ganz besonders bevorzugt sind Oxidkeramiken auf Basis von tetragonalem Zirkonoxid-Polykristall (TZP), die in geeigneter Weise zum Beispiel durch $Y_2O_3$, $CeO_2$, MgO und/oder CaO stabilisiert sind. Insbesondere bevorzugte Oxidkeramiken sind Yttriumoxid-stabilisierter tetragonaler Zirkonoxid-Polykristall (Y-TZP), Ceroxid-stabilisierter tetragonaler Zirkonoxid-Polykristall (Ce-TZP), Zirkonoxid-verstärktes Aluminiumoxid (ZTA) und Aluminiumoxid-verstärktes Zirkonoxid (ATZ).

Es insbesondere bevorzugt, dass die Gesamtmenge an $ZrO_2$, $Y_2O_3$ und $HfO_2$ in der Oxidkeramik auf Basis von Zirkonoxid mindestens 99,0 Gew.-% beträgt. Es ist weiter bevorzugt, dass die Oxidkeramik auf Basis von Zirkonoxid die folgenden Komponenten in den angegebenen Mengen enthält:

| | |
|---|---|
| $Y_2O_3$ | 2,0 bis 10,0 Gew.-%, insbesondere 4,5 bis 6,0 Gew.-%, |
| $HfO_2$ | bis zu 5,0 Gew.-%, |
| $Al_2O_3$ | bis zu 5,0 Gew.-%, insbesondere bis zu 0,5 Gew.-%, |
| $SiO_2$ | bis zu 0,1 Gew.-%, und |
| $Na_2O$ | bis zu 0,1 Gew.-%. |

Die oben beschriebenen Rohlinge eignen sich in besonderer Weise zur Herstellung von mehrgliedrigen Dentalrestaurationen. Ganz besonders eigenen sich die Rohlinge zur Herstellung von Dentalrestaurationen, insbesondere von Brücken, die zwei oder mehr Glieder umfassen.

Üblicherweise haben die verschiedenen Schichten des Rohlings unterschiedliche Farben. Im Sinne der vorliegenden Anmeldung beziehen sich die Begriffe "Farbe" und "gefärbt" auf die Farbe, Helligkeit und/oder Transluzenz einer Schicht.

"Transluzenz" ist die Lichtdurchlässigkeit eines Materials, Körpers oder einer Schicht, d.h. das Verhältnis von durchgelassener zu eingestrahlter Lichtintensität.

Farben können auch durch ihre L*a*b-Werte oder durch einen in der Dentalindustrie üblicherweise verwendeten Farbcode charakterisiert werden. Beispiele für solche Farbcodes sind Vitapan classical® und Vita 3D Master®, beide von VITA Zahnfabrik H. Rauter GmbH & Co. KG, und Chromascop® von der Ivoclar Vivadent AG.

Es ist besonders bevorzugt, dass die Farben der unterschiedlichen Schichten im Bereich der Farben natürlicher Zähne liegen.

Die Schichten sind vorzugsweise planare Schichten, die parallel aufeinander angeordnet sind. Der Rohling liegt vorzugsweise in Form eines rechteckigen Blocks, einer Scheibe, eines Zylinders, einer dentalen Vorform, einer Stiftaufbau-Vorform, eines Zahnabschnitts, eines Hufeisens, eines Kegels, eines Kegelsegments, einer Pyramide, eines Pyramidesegments, eines Torus, eines Torussegments, eines konischen Stumpfs, eines konischen Stumpfsegments, einer Röhre, eines Röhrensegments, einer Kugel, eines Kugelsegments, eines Ellipsoids oder eines Ellipsoidsegments, jeweils mit oder ohne Kerbe oder Vorsprung, vor.

In einer weiteren bevorzugten Ausführungsform zeichnet sich der erfindungsgemäß verwendete Rohling dadurch aus, dass er einen Distorsionskoeffizienten

$$d = \frac{(HV_{max} - HV_{min})}{\overline{HV}}$$

von weniger als 0,4, insbesondere weniger als 0,35, insbesondere weniger als 0,3, vorzugsweise weniger 0,25, weiter bevorzugt weniger als 0,2 und am meisten bevorzugt weniger als 0,1 aufweist, wobei der Koeffizient auf Basis von mindestens einer Messung von HV für jede der unterschiedlichen Schichten berechnet wird,

wobei:

HV die Vickers-Härte gemessen bei einer Kraft im Bereich von 2,5 bis 5,0 kgf (24,517 bis 49,034 N) und insbesondere bei einer Kraft von 5,0 kgf (49,034 N) gemäß ISO 14705:2008 ist;
$HV_{max}$ das Maximum der gemessenen Werte von HV ist;
$HV_{min}$ das Minimum der gemessenen Werte von HV ist; und
$\overline{HV}$ das arithmetische Mittel der gemessenen Werte von HV ist.

Dabei ist die Vickers-Härte nach dem Standard ISO 14705:2008 besonders geeignet, um das Sinterverhalten von verschiedenen Bereichen innerhalb des vorgesinterten mehrschichtigen Rohlings vorherzusagen.

Die Berechnung des Distorsionskoeffizienten sollte allgemein auf einer Mehrzahl von Messungen der Vickershärte an Positionen basieren, die jede der unterschiedlichen Schichten des Rohlings erfassen. Es ist auch möglich, Messungen der Vickershärte auf inneren Oberflächen des Rohlings zu erfassen, die zugänglich werden, indem der Rohling in einzelne Abschnitte oder Scheiben geschnitten wird. Es ist weiter bevorzugt, dass die Messungen einen Teil des Rohlings erfassen, der mindestens so groß ist wie eine typische aus dem Rohling herzustellende Restauration.

In einer Ausführungsform wird der Distorsionskoeffizient auf Basis von Messungen von HV an Messpunkten berechnet, die mit konstantem Abstand entlang einer ersten Linie verteilt sind, die die unterschiedlichen Schichten auf einer äußeren Oberfläche des Rohlings schneidet. Vorzugsweise sind zusätzliche Messpunkte mit konstantem Abstand entlang einer zweiten Linie verteilt, die parallel zu der ersten Linie auf einer Oberfläche im Zentrum des Rohlings liegt, welche durch Schneiden des Rohlings zugänglich gemacht worden ist.

Es ist besonders bevorzugt, dass der konstante Abstand zwischen den Messpunkten entlang der ersten und zweiten Linien nicht mehr als 5 mm beträgt. Es ist weiter bevorzugt, dass die Oberfläche im Zentrum des Rohlings dadurch zugänglich gemacht worden ist, dass der Rohling halbiert worden ist.

Die HV-Werte von vorgesinterten Keramikmaterialien für CAD/CAM-Anwendungen liegen typischerweise im Bereich von 300 bis 1000 MPa.

Die Erfindung betrifft auch die Verwendung eines mehrschichtigen Oxidkeramik-Grünkörpers zur Herstellung dentaler Restaurationen, der mindestens zwei unterschiedliche Schichten umfasst, wobei die Oxidkeramik auf Zirkonoxid basiert und wobei mindestens eine Schicht 0,005 bis 1,0 Gew.-% $La_2O_3$ enthält und sich die mindestens zwei unterschiedlichen Schichten in ihrem Gehalt an $La_2O_3$ unterscheiden.

Bevorzugte Ausführungsformen für den Oxidkeramik-Grünkörper sind wie oben für den erfindungsgemäß verwendeten Oxidkeramikrohling definiert.

In einer bevorzugten Ausführungsform zeichnet sich der erfindungsgemäß verwendete Oxidkeramik-Grünkörper dadurch aus, dass er einen Distorsionskoeffizienten

$$d = \frac{(HV_{max} - HV_{min})}{\overline{HV}}$$

von weniger als 0,4, insbesondere weniger als 0,35, insbesondere weniger als 0,3, vorzugsweise weniger 0,25, weiter bevorzugt weniger als 0,2 und am meisten bevorzugt weniger als 0,1 aufweist, wobei der Koeffizient auf Basis von mindestens einer Messung von HV für jede der unterschiedlichen Schichten berechnet wird, nach einem Sinterschritt bei einer Temperatur im Bereich von 850 bis 1350°C, insbesondere 900 bis 1200°C, bevorzugt 950 bis 1150°C, weiter bevorzugt 1000 bis 1100°C und am meisten bevorzugt bei einer Temperatur von etwa 1100°C,

wobei:

HV die Vickers-Härte gemessen bei einer Kraft im Bereich von 2,5 bis 5,0 kgf (24,517 bis 49,034 N) und insbesondere bei einer Kraft von 5,0 kgf (49,034 N) gemäß ISO 14705:2008 ist;

$HV_{max}$ das Maximum der gemessenen Werte von HV ist;

$HV_{min}$ das Minimum der gemessenen Werte von HV ist; und

$\overline{HV}$ das arithmetische Mittel der gemessenen Werte von HV ist.

Bei dem Sinterschritt wird der Grünkörper vorzugsweise mit Heizraten von 1 bis 10 K/min, vorzugsweise 5 K/min, auf eine Temperatur von 50 K unterhalb der gewünschten Sintertemperatur und 1 bis 3 K/min, vorzugsweise 1 K/min, auf die gewünschte Sintertemperatur, vorzugsweise etwa 1100°C, geheizt und bei dieser Temperatur für 30 bis 480 min, vorzugsweise 120 bis 180 min, gehalten. In einer besonderen Ausführungsform wird der Grünkörper mit Heizraten von 5 K/min auf eine Temperatur von 50 K unterhalb der gewünschten Sintertemperatur und 1 K/min bis zur gewünschten Sintertemperatur, vorzugsweise etwa 1100°C, geheizt und bei dieser Temperatur für 120 min gehalten. Wenn der Grünkörper Bindemittel enthält, geht dem Sinterschritt typischerweise ein Entbinderungsschritt voraus, bei dem vorzugsweise mit einer Heizrate von 0,1 bis 0,5 K/min, bevorzugt 0,1 bis 0,3 K/min und weiter bevorzugt 0,25 K/min, bis auf 300°C, 500°C oder 700°C geheizt wird, mit Haltezeiten von 20 bis 120 min, vorzugsweise 60 min, bei 300°C und/oder 500°C und/oder 700°C. In einer besonderen Ausführungsform wird bei dem Entbinderungsschritt mit einer Heizrate von 0,25 K/min bis auf 700°C geheizt, mit Haltezeiten von 60 min bei 300°C, 500°C und 700°C.

Im Sinne dieser Anmeldung bezieht sich der Begriff "Grünkörper" allgemein auf einen ungesinterten Keramikkörper, der typischerweise durch Verdichten, wie z.B. Verpressen, von Oxidkeramikpulver hergestellt worden ist.

Vorzugsweise ist das Sinterverhalten der mindestens zwei unterschiedlichen Schichten des erfindungsgemäß verwendeten Rohlings oder des erfindungsgemäß verwendeten Grünkörpers so angeglichen, dass der Rohling oder Grünkörper verzugsfrei sintern kann.

Weiterhin betrifft die Erfindung ein Verfahren zur Herstellung des oben beschriebenen Rohlings oder Grünkörpers. Dieses Verfahren zeichnet sich dadurch aus, dass

(a) mindestens ein erstes Oxidkeramikmaterial und ein zweites Oxidkeramikmaterial bereitgestellt werden, die sich in ihrer chemischen Zusammensetzung unterscheiden,
(b) mindestens einem der Oxidkeramikmaterialien $La_2O_3$ zugefügt wird und
(c) gegebenenfalls mindestens einem der Oxidkeramikmaterialien $Al_2O_3$ und/oder MgO zugefügt wird.

Dabei ist es weiter bevorzugt, dass mindestens eines der Oxidkeramikmaterialien und vorzugsweise beide Oxidkeramikmaterialien $Y_2O_3$ enthalten, wobei es besonders bevorzugt ist, dass sich das erste Oxidkeramikmaterial und das zweite Oxidkeramikmaterial in ihrem Gehalt an $Y_2O_3$ unterscheiden.

In einer bevorzugten Ausführungsform des erfindungsgemäßen Verfahrens wird mindestens ein Oxidkeramikmaterial mit $La_2O_3$ infiltriert und/oder mindestens ein Oxidkeramikmaterial mit $Al_2O_3$ und/ oder MgO infiltriert. Geeignete Infiltrationsverfahren sind beispielsweise in der US 2014/135200 A1 beschrieben.

In einer anderen bevorzugten Ausführungsform des erfindungsgemäßen Verfahrens wird mindestens ein Oxidkeramikmaterial mit $La_2O_3$ beschichtet und/oder mindestens ein Oxidkeramikmaterial mit $Al_2O_3$ und/oder MgO beschichtet. Geeignete Beschichtungsverfahren sind beispielsweise in der US 2007/292597 A1 beschrieben.

Gemäß einer weiter bevorzugten Ausführungsform zeichnet sich das erfindungsgemäße Verfahren ferner dadurch aus, dass

(d) Schichten der Oxidkeramikmaterialien gebildet und die Schichten aufeinander angeordnet werden,
(e) die Oxidkeramikmaterialien kompaktiert und insbesondere verpresst werden, um den Grünkörper zu erhalten, und
(f) gegebenenfalls der Grünkörper vorgesintert wird, um den vorgesinterten Keramikrohling zu erhalten.

In einer ganz besonders bevorzugten Ausführungsform weisen die Schichten der Oxidkeramikmaterialien eine kontinuierliche Änderung der Zusammensetzung von der Zusammensetzung des ersten Oxidkeramikmaterials zur Zusammensetzung des zweiten Oxidkeramikmaterials auf. Dies ermöglicht die Bildung eines stufenlosen Farbverlaufs ohne sichtbare Grenzen und Übergänge zwischen einzelnen Schichten.

Die Erfindung betrifft schließlich auch die Verwendung eines mehrschichtigen Oxidkeramikrohlings oder Grünkörpers, der nach dem erfindungsgemäßen Verfahren erhältlich ist. Bevorzugte Ausführungsformen des Oxidkeramikrohlings oder Grünkörpers sind wie oben für den Oxidkeramikrohling beschrieben.

Die mehrschichtigen Oxidkeramikkörper eignen sich besonders zur Herstellung von Dentalrestaurationen. Daher betrifft die Erfindung auch Verfahren zur Herstellung einer Dentalrestauration, bei dem ein wie oben beschriebener Rohling oder Grünkörper verwendet wird.

Bei dem Verfahren zur Herstellung einer Dentalrestauration wird dem mehrschichtigen Oxidkeramikkörper vorzugsweise die Form einer gewünschten Geometrie gegeben, um ein geformtes Keramikprodukt zu erhalten. Es ist bevorzugt, dass die Formgebung durch maschinelle Bearbeitung durchgeführt wird. Die maschinelle Bearbeitung wird typischerweise durch einen Computer gesteuert, vorzugsweise unter Verwendung eines CAD/CAM-Verfahrens.

In einer bevorzugten Ausführungsform weist das geformte Keramikprodukt die Form eines dentalen Gerüsts oder Stiftaufbaus oder einer monolithischen vollanatomischen Dentalrestauration, insbesondere einer mehrgliedrigen Dentalrestauration, auf.

Es ist weiter bevorzugt, dass das geformte Keramikprodukt ferner dichtgesintert wird.

Die Erfindung wird im Folgenden anhand von Beispielen näher erläutert.

**Beispiele**

**Allgemeines Verfahren zur Behandlung von Oxidpulvern mit Färbemitteln und/oder Dotierungsmitteln**

Die Behandlung von Oxidpulvern in den folgenden Beispielen wurde analog zu US 2014/135200 A1 durchgeführt.

Hierzu wurde eine wässrige Behandlungslösung hergestellt, die eine geeignete Menge wasserlöslicher Nitrate der Elemente enthielt, mit denen das Oxidpulver behandelt werden sollte. Eine geeignete Menge Oxidpulver (beispielsweise 1.000 g) wurde in den Mischbehälter eines Labormischers "EL1" (Fa. Eirich, Hardheim) eingebracht, der ferner ein Rührwerkzeug (Sternwirbler) und eine Sprühdüse (0,3 mm Hohlkegel) zum Aufbringen einer Lösung aufwies. Der Mischbehälter wurde mit einer Geschwindigkeit von 13 m/s in Bewegung versetzt und das darin befindliche Oxidpulver durch das Rührwerkzeug gleichmäßig verwirbelt. Dann wurden je 1 g Oxidpulver etwa 0,1 g Behandlungslösung mit Hilfe einer Schlauchpumpe vom Typ 120S/DV (Fa. Watson Marlow, Rommerskirchen; Drehzahl 170 U/min) über die Sprühdüse auf das Oxidpulver aufgebracht und so das Oxidpulver gleichmäßig mit der Behandlungslösung infiltriert.

**Beispiel 1**

*Anpassung des Sinterverhaltens eines zweischichtigen Rohlings aus Zirkonoxidpulvern mit unterschiedlichem Yttrium-Gehalt durch Dotierung mit $La_2O_3$*

Zur Herstellung von eingefärbten Zirkonoxidpulvern wurden kommerziell erhältliche Zirkonoxidpulver (TOSOH TZ-PX-471 und TOSOH Zpex Smile) als Rohstoffe eingesetzt und gemäß der folgenden Tabelle unter Verwendung des allgemeinen Verfahrens mit Lösungen von Nitratsalzen von färbenden Elementen und gegebenenfalls Lanthan als Dotierungsmittel behandelt. Dadurch wurden ein für eine Dentinschicht geeignetes Pulver (L1) und ein für eine Schneideschicht geeignetes Pulver (L2) erhalten:

| Pulver | Ausgangspulver | Färbende Elemente[1] | Dotierungsmittel |
|---|---|---|---|
| L2 Schneide | TOSOH Zpex Smile[2] | 0,028 Gew.-% Fe[4]<br>0,0014 Gew.-% Cr[5]<br>0,2283 Gew.-% Er[7] | - |
| L1 Dentin | TOSOH TZ-PX-471[3] | 0,0091 Gew.-% Fe[4]<br>0,0024 Gew.-% Cr[5]<br>0,002 Gew.-% Pr[6]<br>0,3981 Gew.-% Er[7] | 0,1537 Gew.-% La[8] |

[1] auf Basis des Gesamtgewichts der Oxidmischung nach dem Sintern
[2] enthält 9,25 Gew.-% $Y_2O_3$ und 0,048 Gew.-% $Al_2O_3$
[3] enthält 7,37 Gew.-% $Y_2O_3$ und 0,048 Gew.-% $Al_2O_3$
[4] berechnet als $Fe_2O_3$
[5] berechnet als $Cr_2O_3$
[6] berechnet als $Pr_2O_3$
[7] berechnet als $Er_2O_3$
[8] berechnet als $La_2O_3$

Anschließend wurden jeweils etwa 10 g der eingefärbten Zirkonoxidpulver nacheinander in zwei Schichten (unten: L1, oben: L2) in das Presswerkzeug (Durchmesser ca. 40 mm) einer Labor-Axialpresse eingefüllt und unter einem Druck von etwa 160 MPa axial verdichtet. Der so erhaltene Grünkörper wurde mit folgendem Brennprogramm entbindert und vorgesintert:

60 K/min bis 120°C,
24 K/min bis 200°C,
10 K/min bis 320°C,
60 K/min bis 1050°C, Haltezeit 3 h.

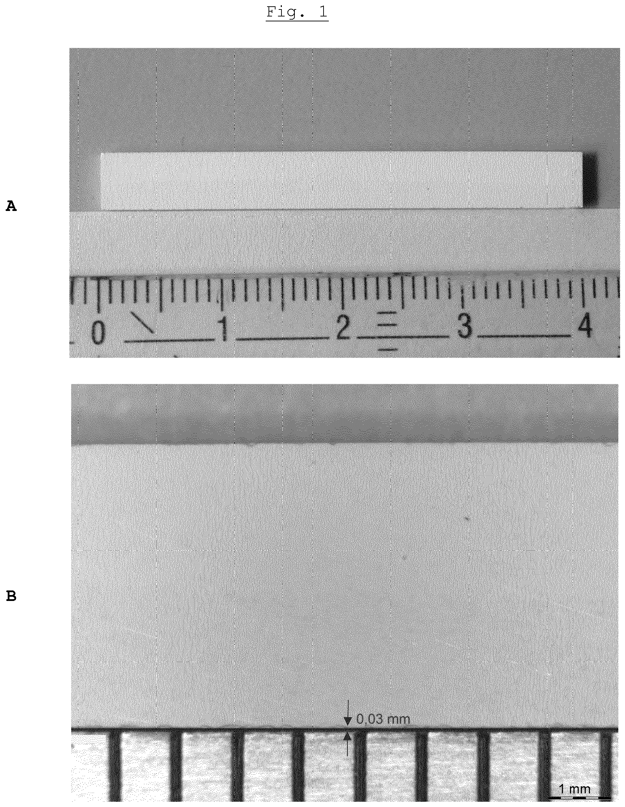

Aus dem so erhaltenen entbinderten und vorgesinterten Rohling wurde mit einer Säge (IsoMet 4000, Fa. Buehler, Esslingen) ein ca. 2 mm dicker Querschnitt herausgesägt. An die beiden unteren Außenkanten des Querschnitts wurde ein Papier bzw. Lineal als Referenzlinie angelegt und unter einem Stereomikroskop (SZX 16, Fa. Olympus, Hamburg) der Bereich der maximalen Durchbiegung vermessen. Die Ergebnisse sind in Fig. 1A (Übersicht) und 1B (Detailansicht) gezeigt. Der vorgesinterte Rohling zeigte lediglich eine vernachlässigbare Durchbiegung von 0,03 mm.

Abschließend wurde der Querschnitt des vorgesinterten Rohlings in einem Brennofen Programat S1 (Ivoclar Vivadent AG, Schaan) mit folgendem Brenn- und Abkühlprogramm dichtgesintert:

600 K/h bis 900°C, Haltezeit 0,5 h
200 K/h bis 1500°C, Haltezeit 2 h
600 K/h bis 900°C,
500 K/h bis 300°C.

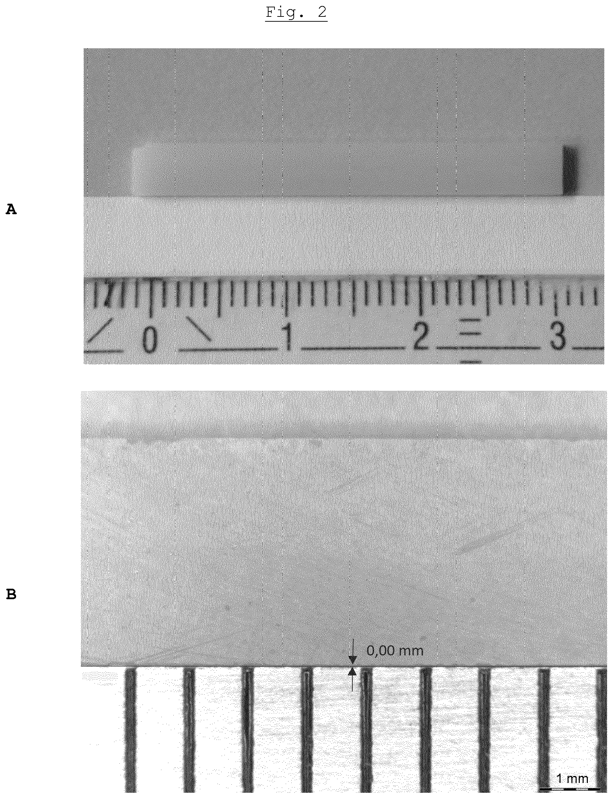

Der Querschnitt des dichtgesinterten Rohlings wurde wiederum wie oben beschrieben unter dem Stereomikroskop vermessen. Die Ergebnisse sind in Fig. 2A (Übersicht) und 2B (Detailansicht) gezeigt. Der dichtgesinterte Rohling zeigte keine messbare Durchbiegung.

**Beispiel 2 (Vergleich)**

*Sinterverhalten eines zweischichtigen Rohlings aus Zirkonoxidpulvern mit unterschiedlichem Yttrium-Gehalt ohne Dotierung mit $La_2O_3$*

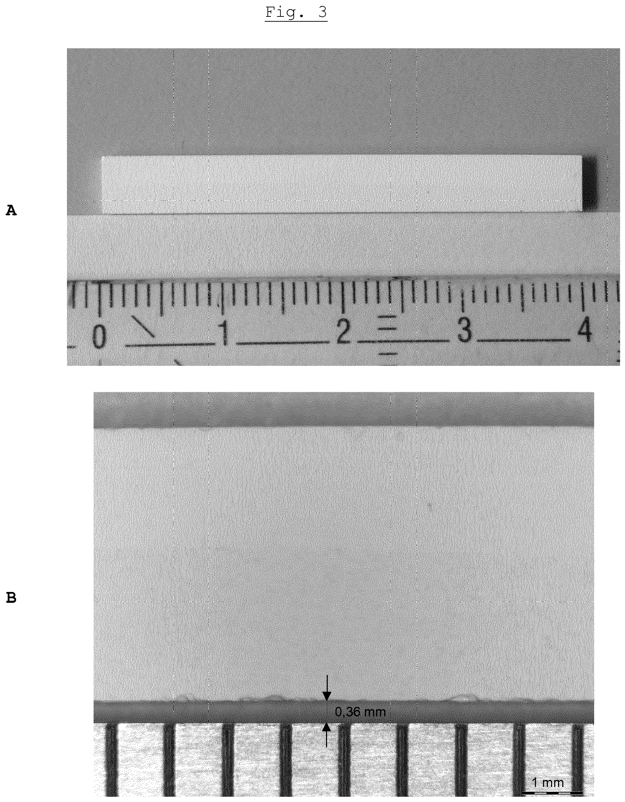

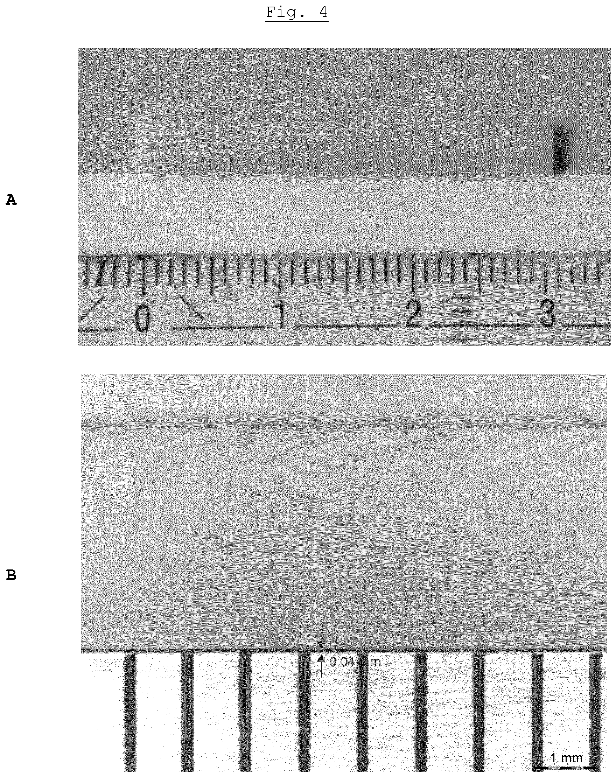

Beispiel 1 wurde identisch wiederholt, wobei jedoch kein Lanthan als Dotierungsmittel zugegeben wurde. Die Ergebnisse für den entbinderten und vorgesinterten Rohling sind in Fig. 3A (Übersicht) und 3B (Detailansicht) gezeigt. Darin ist deutlich zu erkennen, dass die Dentinschicht (L1) stärker geschrumpft ist als die Schneideschicht (L2), wodurch der Rohling eine konvexe Form erhalten hat. Die Durchbiegung des Rohlings betrug 0,36 mm. Die Ergebnisse nach dem Dichtsintern des Rohlings sind in Fig. 4A (Übersicht) und 4B (Detailansicht) gezeigt. Auch der dichtgesinterte Rohling zeigte danach noch eine Durchbiegung von 0,04 mm.

**Beispiel 3**

*Anpassung des Sinterverhaltens eines vierschichtigen Rohlings aus Zirkonoxidpulvern mit unterschiedlichem Yttrium-Gehalt durch Dotierung mit $La_2O_3$*

Zur Herstellung von eingefärbten Zirkonoxidpulvern wurden kommerziell erhältliche Zirkonoxidpulver (TOSOH TZ-PX-471 und TOSOH Zpex Smile) als Rohstoffe eingesetzt und gemäß der folgenden Tabelle unter Verwendung des allgemeinen Verfahrens mit Lösungen von Nitratsalzen von färbenden Elementen und gegebenenfalls Lanthan als Dotierungsmittel behandelt. Dadurch wurden ein für eine Dentinschicht geeignetes Pulver (L1) und ein für eine Schneideschicht geeignetes Pulver (L4) erhalten. Durch Vermischen dieser Pulver im Verhältnis 1:2 bzw. 2:1 mittels eines Schüttelmischers (Turbula, Fa. WAB, Muttenz) wurden zudem zwei für Zwischenschichten geeignete Pulver erhalten:

| Pulver | Ausgangspulver | Färbende Elemente[1] | Dotierungsmittel |
|---|---|---|---|
| L4 Schneide | TOSOH Zpex Smile[2] | 0,028 Gew.-% Fe[4]  0,0014 Gew.-% Cr[5]  0,2283 Gew.-% Er[7] | - |

(fortgesetzt)

| Pulver | Ausgangspulver | Färbende Elemente[1] | Dotierungsmittel |
|---|---|---|---|
| L3 Zwischenschicht 2 | Mischung aus TOSOH Zpex Smile[2] und TOSOH TZ-PX-471[3] (2:1) | 0,0489 Gew.-% Fe[4] <br> 0,0017 Gew.-% Cr[5] <br> 0,0007 Gew.-% Pr[6] <br> 0,2849 Gew.-% Er[7] | 0,0512 Gew.-% La[8] |
| L2 | Mischung aus | 0,0698 Gew.-% Fe[4] | 0,1025 Gew.-% La[8] |
| Zwischenschicht 1 | TOSOH Zpex Smile[2] und TOSOH TZ-PX-471[3] (1:2) | 0,0021 Gew.-% Cr[5] <br> 0,0014 Gew.-% Pr[6] <br> 0,3415 Gew.-% Er[7] | |
| L1 Dentin | TOSOH TZ-PX-471[3] | 0,0091 Gew.-% Fe[4] <br> 0,0024 Gew.-% Cr[5] <br> 0,002 Gew.-% Pr[6] <br> 0,3981 Gew.-% Er[7] | 0,1537 Gew.-% La[8] |
| [1] auf Basis des Gesamtgewichts der Oxidmischung nach dem Sintern <br> [2] enthält 9,25 Gew.-% $Y_2O_3$ und 0,048 Gew.-% $Al_2O_3$ <br> [3] enthält 7,37 Gew.-% $Y_2O_3$ und 0,048 Gew.-% $Al_2O_3$ <br> [4] berechnet als $Fe_2O_3$ <br> [5] berechnet als $Cr_2O_3$ <br> [6] berechnet als $Pr_2O_3$ <br> [7] berechnet als $Er_2O_3$ <br> [8] berechnet als $La_2O_3$ | | | |

Anschließend wurden jeweils etwa 19 g der eingefärbten Zirkonoxidpulver nacheinander in vier Schichten (unten: L1, oben: L4) in das Presswerkzeug (Durchmesser ca. 40 mm) einer Labor-Axialpresse eingefüllt und unter einem Druck von etwa 160 MPa axial verdichtet. Der so erhaltene Grünkörper wurde mit folgendem Brennprogramm entbindert und vorgesintert:

60 K/min bis 120°C,
24 K/min bis 200°C,
10 K/min bis 320°C,
60 K/min bis 1050°C, Haltezeit 3 h.

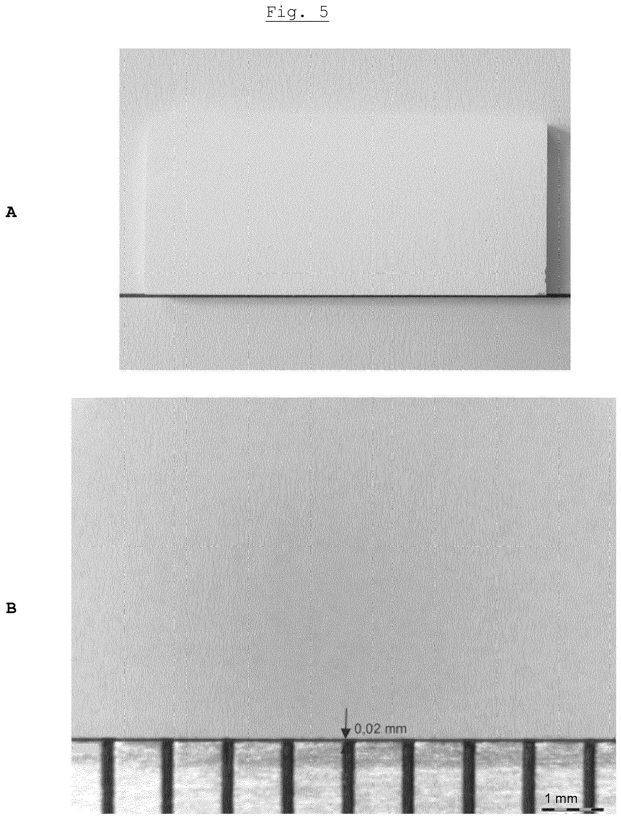

Aus dem so erhaltenen entbinderten und vorgesinterten Rohling wurde mit einer Säge (IsoMet 4000, Fa. Buehler, Esslingen) ein ca. 2 mm dicker Querschnitt herausgesägt. An die beiden unteren Außenkanten des Querschnitts wurde ein Papier bzw. Lineal als Referenzlinie angelegt und unter einem Stereomikroskop (SZX 16, Fa. Olympus, Hamburg) der Bereich der maximalen Durchbiegung vermessen. Die Ergebnisse sind in Fig. 5A (Übersicht) und 5B (Detailansicht) gezeigt. Der vorgesinterte Rohling zeigte lediglich eine vernachlässigbare Durchbiegung von 0,02 mm.

Außerdem wurde an dem Querschnitt des vorgesinterten Rohlings der Verlauf der Vickershärte HV$_5$ über die Schichten an 10 Messpunkten im Abstand von jeweils 1,5 mm mit einem Härteprüfer (ZHU 0.2, Fa. Zwick Roell, Ulm) gemessen. Die ermittelten Härtewerte sind in der folgenden Tabelle aufgeführt:

| Messpunkt | HV$_5$ [MPa] |
|-----------|--------------|
| 1 | 583 |
| 2 | 591 |
| 3 | 593 |
| 4 | 584 |
| 5 | 586 |
| 6 | 652 |
| 7 | 623 |
| 8 | 636 |
| 9 | 605 |
| 10 | 597 |

Aus diesen Werten wurde ein Distorsionskoeffizient von d = 0,114 errechnet.

Abschließend wurde der Rohling in einem Brennofen Programat S1 (Ivoclar Vivadent AG, Schaan) mit folgendem Brenn- und Abkühlprogramm dichtgesintert:

> 600 K/h bis 900°C, Haltezeit 0,5 h
> 200 K/h bis 1500°C, Haltezeit 2 h
> 600 K/h bis 900°C,
> 500 K/h bis 300°C.

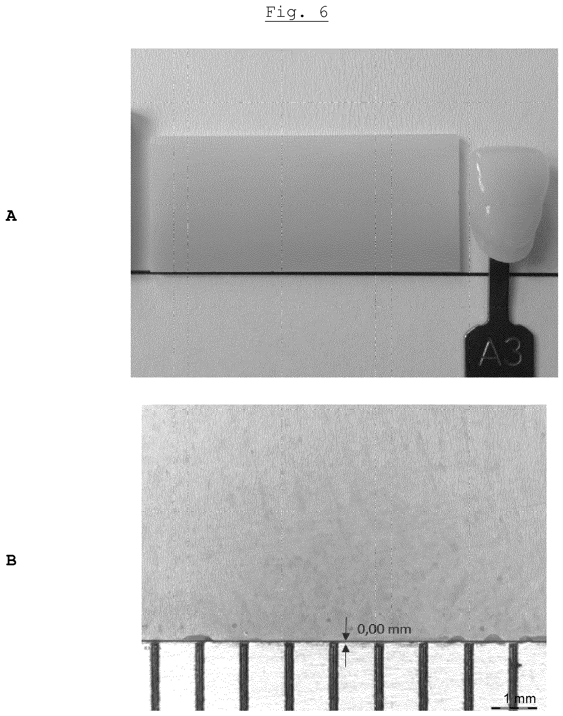

Aus dem so erhaltenen dichtgesinterten Rohling wurde wiederum wie oben beschrieben ein ca. 2 mm dicker Querschnitt herausgesägt und unter dem Stereomikroskop vermessen. Die Ergebnisse sind in Fig. 6A (Übersicht) und 6B (Detailansicht) gezeigt. Der dichtgesinterte Rohling zeigte keine messbare Durchbiegung.

### Beispiel 4

*Anpassung des Sinterverhaltens eines vierschichtigen Rohlings aus Zirkonoxidpulvern mit unterschiedlichem Yttrium-Gehalt durch Dotierung mit La$_2$O$_3$*

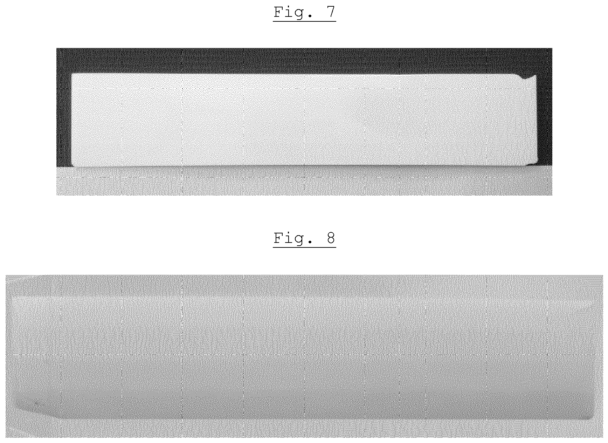

Beispiel 3 wurde mit einem größeren Ansatz wiederholt. Dazu wurden jeweils etwa 115 g der wie in Beispiel 3 erhaltenen eingefärbten Zirkonoxidpulver nacheinander in vier Schichten (unten: L1, oben: L4) in das Presswerkzeug (Durchmesser ca. 100 mm) einer Pulverpresse eingefüllt und unter einem Druck von etwa 160 MPa axial verdichtet. Die Rohlinge wurden wie in Beispiel 3 beschrieben zunächst entbindert und vorgesintert und anschließend dichtgesintert. Aus den so erhaltenen Rohlingen wurden jeweils ca. 2 mm dicke Querschnitte herausgesägt und unter dem Stereomikroskop vermessen. Die Ergebnisse sind in Fig. 7 (entbinderter und vorgesinterter Rohling) und Fig. 8 (dichtgesinterter Rohling im Durchlicht) gezeigt. Die Rohlinge zeigten keine messbare Durchbiegung.

Außerdem wurde an dem Querschnitt des vorgesinterten Rohlings der Verlauf der Vickershärte HV$_5$ über die Schichten an 10 Messpunkten im Abstand von jeweils 2 mm mit einem Härteprüfer (ZHU 0.2, Fa. Zwick Roell, Ulm) gemessen. Die ermittelten Härtewerte sind in der folgenden Tabelle aufgeführt:

| Messpunkt | HV$_5$ [MPa] |
|-----------|--------------|
| 1 | 581 |
| 2 | 588 |
| 3 | 599 |

(fortgesetzt)

| Messpunkt | HV$_5$ [MPa] |
|---|---|
| 4 | 600 |
| 5 | 589 |
| 6 | 625 |
| 7 | 634 |
| 8 | 620 |
| 9 | 606 |
| 10 | 613 |

Aus diesen Werten wurde ein Distorsionskoeffizient von d = 0,088 errechnet.

**Beispiel 5**

*Anpassung des Sinterverhaltens eines Rohlings mit kontinuierlichem Farb- und Transluzenzverlauf aus Zirkonoxidpulvern mit unterschiedlichem Yttrium-Gehalt durch Dotierung mit* La$_2$O$_3$

Zur Herstellung von eingefärbten Zirkonoxidpulvern wurden kommerziell erhältliche Zirkonoxidpulver (TOSOH TZ-PX-471 und TOSOH Zpex Smile) als Rohstoffe eingesetzt und gemäß der folgenden Tabelle unter Verwendung des allgemeinen Verfahrens mit Lösungen von Nitratsalzen von färbenden Elementen und gegebenenfalls Lanthan als Dotierungsmittel behandelt. Dadurch wurden ein für eine Dentinschicht geeignetes Pulver (L1) und ein für eine Schneideschicht geeignetes Pulver (L2) erhalten:

| Pulver | Ausgangspulver | Färbende Elemente[1] | Dotierungsmittel |
|---|---|---|---|
| L2 Schneide | TOSOH Zpex Smile[2] | 0,065 Gew.-% Fe[4]<br>0,00025 Gew.-% Mn[5]<br>0,0005 Gew.-% Pr[6]<br>0,0108 Gew.-% Tb[7]<br>0,24 Gew.-% Er[8] | - |
| L1 Dentin | TOSOH TZ-PX-471[3] | 0,092 Gew.-% Fe[4]<br>0,00063 Gew.-% Mn[5]<br>0,0008 Gew.-% Pr[6]<br>0,0108 Gew.-% Tb[7]<br>0,5 Gew.-% Er[8] | 0,13 Gew.-% La[9] |
| [1] auf Basis des Gesamtgewichts der Oxidmischung nach dem Sintern<br>[2] enthält 9,25 Gew.-% Y$_2$O$_3$ und 0,048 Gew.-% Al$_2$O$_3$<br>[3] enthält 7,37 Gew.-% Y$_2$O$_3$ und 0,048 Gew.-% Al$_2$O$_3$<br>[4] berechnet als Fe$_2$O$_3$<br>[5] berechnet als Mn$_2$O$_3$<br>[6] berechnet als Pr$_2$O$_3$<br>[7] berechnet als Tb$_2$O$_3$<br>[8] berechnet als Er$_2$O$_3$<br>[9] berechnet als La$_2$O$_3$ | | | |

Anschließend wurden die eingefärbten Zirkonoxidpulver mit einem geeigneten Füllverfahren in Form eines kontinuierlichen Gradienten in das Presswerkzeug (Durchmesser ca. 100 mm) einer Pulverpresse eingefüllt und unter einem Druck von etwa 160 MPa axial verdichtet. Der so erhaltene Grünkörper wurde mit folgendem Brennprogramm entbindert und vorgesintert:

60 K/min bis 120°C,

24 K/min bis 200°C,
10 K/min bis 320°C,
60 K/min bis 1050°C, Haltezeit 3 h.

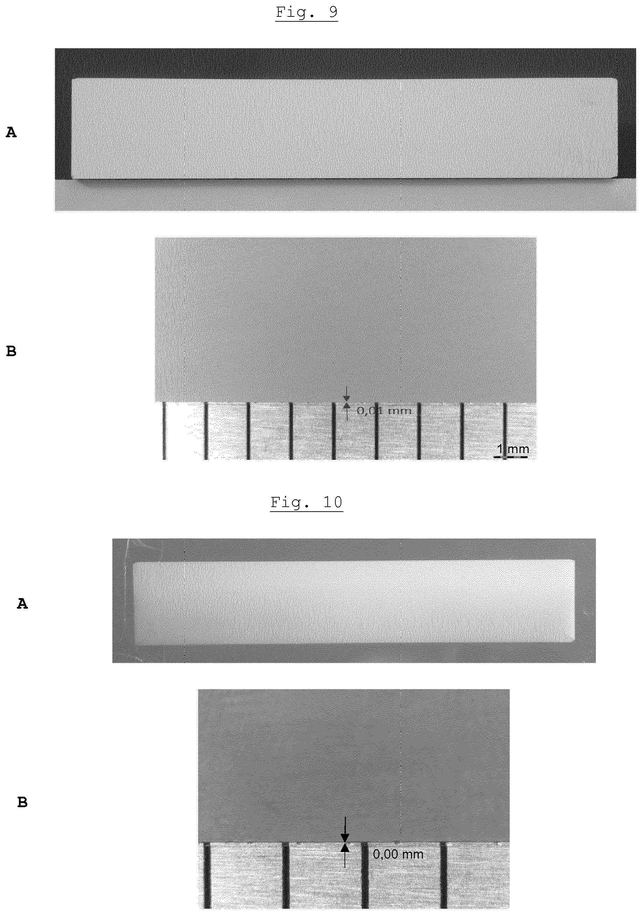

Aus dem so erhaltenen entbinderten und vorgesinterten Rohling wurde mit einer Säge (IsoMet 4000, Fa. Buehler, Esslingen) ein ca. 2 mm dicker Querschnitt herausgesägt. An die beiden unteren Außenkanten des Querschnitts wurde ein Papier bzw. Lineal als Referenzlinie angelegt und unter einem Stereomikroskop (SZX 16, Fa. Olympus, Hamburg) der Bereich der maximalen Durchbiegung vermessen. Die Ergebnisse sind in Fig. 9A (Übersicht) und 9B (Detailansicht) gezeigt. Der vorgesinterte Rohling zeigte lediglich eine vernachlässigbare Durchbiegung von 0,01 mm.

Außerdem wurde an dem Querschnitt des vorgesinterten Rohlings der Verlauf der Vickershärte $HV_5$ über den Gradienten an 10 Messpunkten im Abstand von jeweils 1,5 mm mit einem Härteprüfer (ZHU 0.2, Fa. Zwick Roell, Ulm) gemessen. Die ermittelten Härtewerte sind in der folgenden Tabelle aufgeführt:

| Messpunkt | $HV_5$ [MPa] |
|-----------|--------------|
| 1 | 732 |
| 2 | 766 |
| 3 | 780 |
| 4 | 724 |
| 5 | 718 |
| 6 | 744 |
| 7 | 756 |
| 8 | 773 |
| 9 | 754 |
| 10 | 724 |

Aus diesen Werten wurde ein Distorsionskoeffizient von d = 0,083 errechnet.

Abschließend wurde der Querschnitt des vorgesinterten Rohlings in einem Brennofen Programat S1 (Ivoclar Vivadent AG, Schaan) mit folgendem Brenn- und Abkühlprogramm dichtgesintert:

600 K/h bis 900°C, Haltezeit 0,5 h
200 K/h bis 1500°C, Haltezeit 2 h
600 K/h bis 900°C,
500 K/h bis 300°C.

Der Querschnitt des dichtgesinterten Rohlings wurde wiederum wie oben beschrieben unter dem Stereomikroskop vermessen. Die Ergebnisse sind in Fig. 10A (Übersicht) und 10B (Detailansicht) gezeigt. Der dichtgesinterte Rohling zeigte keine messbare Durchbiegung.

**Beispiel 6**

*Anpassung des Sinterverhaltens eines zweischichtigen Rohlings aus Zirkonoxidpulvern mit unterschiedlichem Yttrium-Gehalt durch Dotierung mit $La_2O_3$, $Al_2O_3$ und MgO*

Zur Herstellung von eingefärbten Zirkonoxidpulvern wurden kommerziell erhältliche Zirkonoxidpulver (TOSOH Zpex und TOSOH Zpex Smile) als Rohstoffe eingesetzt und gemäß der folgenden Tabelle unter Verwendung des allgemeinen Verfahrens mit Lösungen von Nitratsalzen von färbenden Elementen und gegebenenfalls Lanthan bzw. Aluminium und Magnesium als Dotierungsmittel behandelt. Dadurch wurden ein für eine Dentinschicht geeignetes Pulver (L1) und ein für eine Schneideschicht geeignetes Pulver (L2) erhalten:

| Pulver | Ausgangspulver | Färbende Elemente[1] | Dotierungsmittel |
|---|---|---|---|
| L2 Schneide | TOSOH Zpex Smile[2] | 0,0676 Gew.-% Fe[4]<br>0,0003 Gew.-% Mn[5]<br>0,0005 Gew.-% Pr[6]<br>0,0112 Gew.-% Tb[7]<br>0,2249 Gew.-% Er[8] | 0,03 Gew.-% Al[9]<br>0,01 Gew.-% Mg[10] |
| L1 Dentin | TOSOH Zpex[3] | 0,0734 Gew.-% Fe[4]<br>0,0006 Gew.-% Mn[5]<br>0,0001 Gew.-% Pr[6]<br>0,0095 Gew.- % Tb[7]<br>0, 6647 Gew.-% Er[8] | 0,62 Gew.-% La[11] |

[1] auf Basis des Gesamtgewichts der Oxidmischung nach dem Sintern
[2] enthält 9,25 Gew.-% $Y_2O_3$ und 0,048 Gew.-% $Al_2O_3$
[3] enthält 5,36 Gew.-% $Y_2O_3$ und 0,048 Gew.-% $Al_2O_3$
[4] berechnet als $Fe_2O_3$
[5] berechnet als $Mn_2O_3$
[6] berechnet als $Pr_2O_3$
[7] berechnet als $Tb_2O_3$
[8] berechnet als $Er_2O_3$
[9] berechnet als $Al_2O_3$
[10] berechnet als $MgO$
[11] berechnet als $La_2O_3$

Anschließend wurden jeweils etwa 10 g der eingefärbten Zirkonoxidpulver nacheinander in zwei Schichten (unten: L1, oben: L2) in das Presswerkzeug (Durchmesser ca. 40 mm) einer Labor-Axialpresse eingefüllt und unter einem Druck von etwa 160 MPa uniaxial verdichtet. Der so erhaltene Grünkörper wurde mit folgendem Brennprogramm entbindert und vorgesintert:

60 K/min bis 120°C,
24 K/min bis 200°C,
10 K/min bis 320°C,
60 K/min bis 1010°C, Haltezeit 3 h.

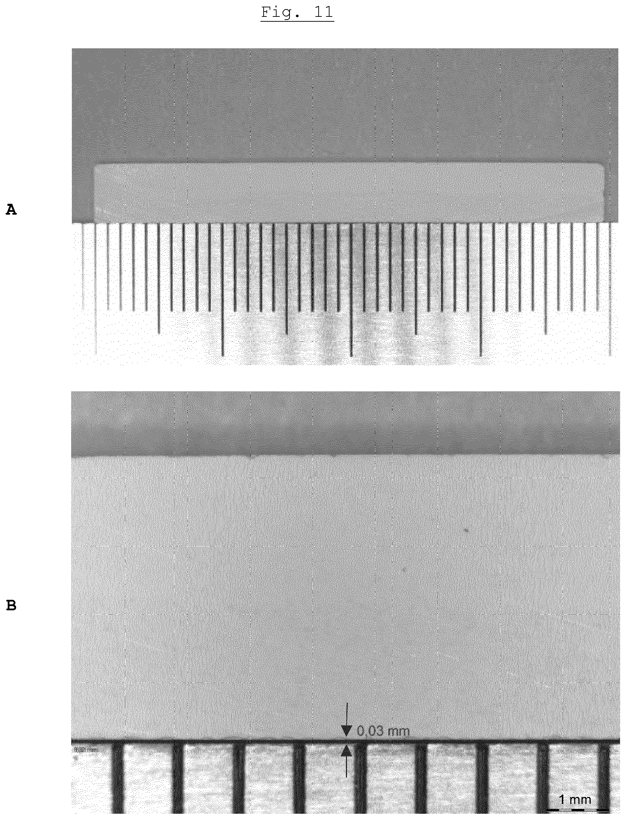

Aus dem so erhaltenen entbinderten und vorgesinterten Rohling wurde mit einer Säge (IsoMet 4000, Fa. Buehler, Esslingen) ein ca. 2 mm dicker Querschnitt herausgesägt. An die beiden unteren Außenkanten des Querschnitts wurde ein Lineal als Referenzlinie angelegt und unter einem Stereomikroskop (SZX 16, Fa. Olympus, Hamburg) der Bereich der maximalen Durchbiegung vermessen. Die Ergebnisse sind in Fig. 11A (Übersicht) und 11B (Detailansicht) gezeigt. Der vorgesinterte Rohling zeigte lediglich eine vernachlässigbare Durchbiegung von 0,03 mm.
Abschließend wurde der Querschnitt des vorgesinterten Rohlings in einem Brennofen Programat S1 (Ivoclar Vivadent AG, Schaan) mit folgendem Brenn- und Abkühlprogramm dichtgesintert:

600 K/h bis 900°C, Haltezeit 0,5 h
200 K/h bis 1500°C, Haltezeit 2 h
600 K/h bis 900°C,
500 K/h bis 300°C.

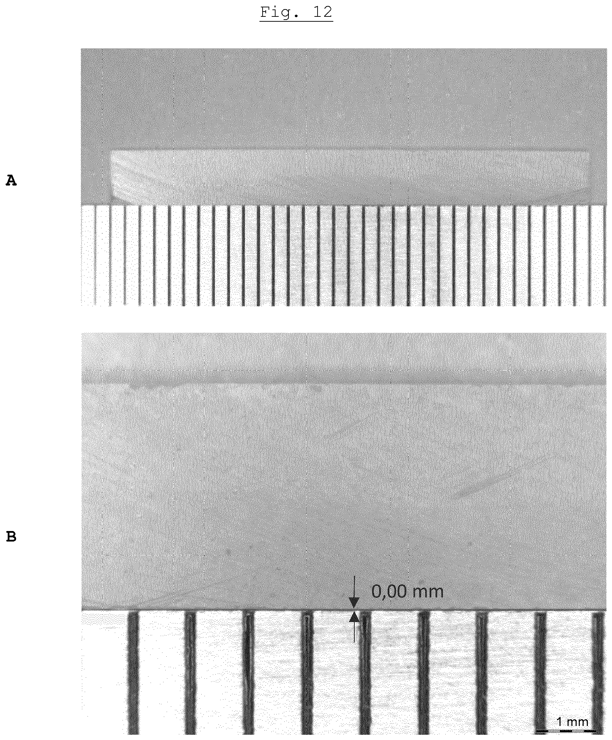

Der Querschnitt des dichtgesinterten Rohlings wurde wiederum wie oben beschrieben unter dem Stereomikroskop vermessen. Die Ergebnisse sind in Fig. 12A (Übersicht) und 12B (Detailansicht) gezeigt. Der dichtgesinterte Rohling zeigte keine messbare Durchbiegung.

**Beispiel 7 (Vergleich)**

*Sinterverhalten eines zweischichtigen Rohlings aus Zirkonoxidpulvern mit unterschiedlichem Yttrium-Gehalt ohne Dotierung mit La$_2$O$_3$*

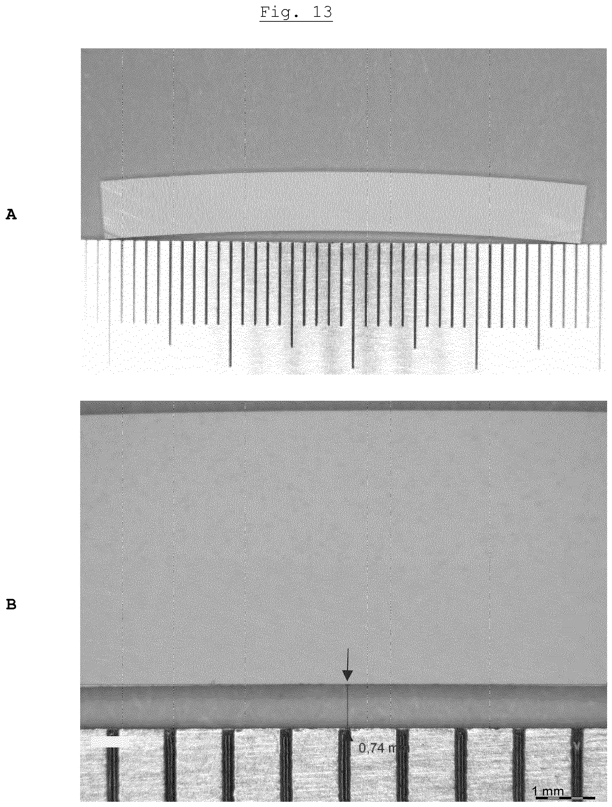

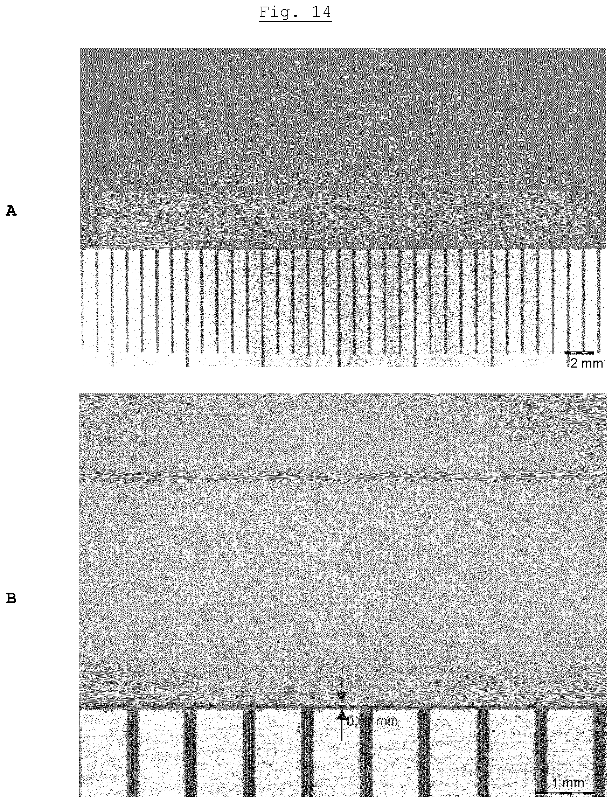

Beispiel 6 wurde identisch wiederholt, wobei jedoch keine Dotierungsmittel (Lanthan, Aluminium, Magnesium) zugegeben wurde. Die Ergebnisse für den entbinderten und vorgesinterten Rohling sind in Fig. 13A (Übersicht) und 13B (Detailansicht) gezeigt. Darin ist deutlich zu erkennen, dass die Dentinschicht (L1) stärker geschrumpft ist als die Schneideschicht (L2), wodurch der Rohling eine konvexe Form erhalten hat. Die Durchbiegung des Rohlings betrug 0,74 mm. Die Ergebnisse nach dem Dichtsintern des Rohlings sind in Fig. 14A (Übersicht) und 14B (Detailansicht) gezeigt. Auch der dichtgesinterte Rohling zeigte danach noch eine Durchbiegung von 0,05 mm.

**Beispiel 8**

*Anpassung des Sinterverhaltens eines Rohlings mit kontinuierlichem Farb- und Transluzenzverlauf aus Zirkonoxidpulvern mit unterschiedlichem Yttrium-Gehalt durch Dotierung mit La$_2$O$_3$, Al$_2$O$_3$ und MgO*

Zur Herstellung von eingefärbten Zirkonoxidpulvern wurden kommerziell erhältliche Zirkonoxidpulver (TOSOH Zpex und TOSOH Zpex Smile) als Rohstoffe eingesetzt und gemäß der folgenden Tabelle unter Verwendung des allgemeinen Verfahrens mit Lösungen von Nitratsalzen von färbenden Elementen und gegebenenfalls Lanthan bzw. Aluminium und Magnesium als Dotierungsmittel behandelt. Dadurch wurden ein für eine Dentinschicht geeignetes Pulver (L1) und ein für eine Schneideschicht geeignetes Pulver (L2) erhalten:

| Pulver | Ausgangspulver | Färbende Elemente[1] | Dotierungsmittel |
|--------|----------------|----------------------|------------------|
| L2 Schneide | TOSOH Zpex Smile[2] | 0,0676 Gew.-% Fe[4]<br>0,0003 Gew.-% Mn[5]<br>0,0005 Gew.-% Pr[6]<br>0,0112 Gew.-% Tb[7]<br>0,2249 Gew.-% Er[8] | 0,03 Gew.-% Al[9]<br>0,01 Gew.-% Mg[10] |
| L1 Dentin | TOSOH Zpex[3] | 0,0734 Gew.-% Fe[4]<br>0,0006 Gew.-% Mn[5]<br>0,0001 Gew.-% Pr[6]<br>0,0095 Gew.- % Tb[7]<br>0, 6647 Gew.-% Er[8] | 0, 62 Gew.-% La[11] |

[1] auf Basis des Gesamtgewichts der Oxidmischung nach dem Sintern
[2] enthält 9,25 Gew.-% Y$_2$O$_3$ und 0,048 Gew.-% Al$_2$O$_3$
[3] enthält 5,36 Gew.-% Y$_2$O$_3$ und 0,048 Gew.-% Al$_2$O$_3$
[4] berechnet als Fe$_2$O$_3$
[5] berechnet als Mn$_2$O$_3$
[6] berechnet als Pr$_2$O$_3$
[7] berechnet als Tb$_2$O$_3$
[8] berechnet als Er$_2$O$_3$
[9] berechnet als Al$_2$O$_3$
[10] berechnet als MgO
[11] berechnet als La$_2$O$_3$

Anschließend wurden die eingefärbten Zirkonoxidpulver mit einem geeigneten Füllverfahren in Form eines kontinuierlichen Gradienten in das Presswerkzeug (Durchmesser ca. 100 mm) einer Pulverpresse eingefüllt und unter einem Druck von etwa 160 MPa axial verdichtet. Der so erhaltene Grünkörper wurde mit folgendem Brennprogramm entbindert und vorgesintert:

60 K/min bis 120°C,
24 K/min bis 200°C,
10 K/min bis 320°C,
60 K/min bis 1050°C, Haltezeit 3 h.

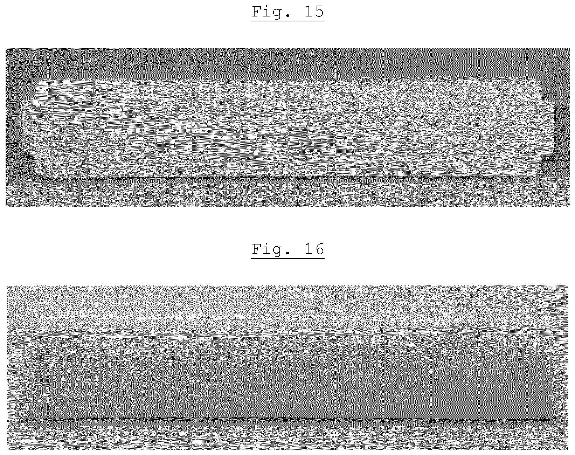

Aus dem so erhaltenen entbinderten und vorgesinterten Rohling wurde mit einer Säge (IsoMet 4000, Fa. Buehler, Esslingen) ein ca. 2 mm dicker Querschnitt herausgesägt. Das Ergebnis ist in Fig. 15 gezeigt. Der vorgesinterte Rohling zeigte lediglich eine vernachlässigbare Durchbiegung.

Außerdem wurde an dem Querschnitt des vorgesinterten Rohlings der Verlauf der Vickershärte $HV_5$ über den Gradienten an 10 Messpunkten im Abstand von jeweils 2 mm mit einem Härteprüfer (ZHU 0.2, Fa. Zwick Roell, Ulm) gemessen. Die ermittelten Härtewerte sind in der folgenden Tabelle aufgeführt:

| Messpunkt | $HV_5$ [MPa] |
|-----------|--------------|
| 1 | 651 |
| 2 | 670 |
| 3 | 676 |
| 4 | 673 |
| 5 | 642 |
| 6 | 600 |
| 7 | 557 |
| 8 | 543 |
| 9 | 560 |
| 10 | 554 |

Aus diesen Werten wurde ein Distorsionskoeffizient von d = 0,22 errechnet.

Abschließend wurde der Querschnitt des vorgesinterten Rohlings in einem Brennofen Programat S1 (Ivoclar Vivadent AG, Schaan) mit folgendem Brenn- und Abkühlprogramm dichtgesintert:

600 K/h bis 900°C, Haltezeit 0,5 h
200 K/h bis 1500°C, Haltezeit 2 h
600 K/h bis 900°C,
500 K/h bis 300°C.

Der Querschnitt des dichtgesinterten Rohlings wurde wiederum wie oben beschrieben unter dem Stereomikroskop vermessen. Das Ergebnis ist in Fig. 16 gezeigt. Der dichtgesinterte Rohling zeigte lediglich eine vernachlässigbare Durchbiegung.

**Patentansprüche**

1. Verwendung eines vorgesinterten mehrschichtigen Oxidkeramikrohlings, der mindestens zwei unterschiedliche Schichten umfasst, wobei die Oxidkeramik auf Zirkonoxid basiert und wobei mindestens eine Schicht 0,005 bis 1,0 Gew.-% $La_2O_3$ enthält und sich die mindestens zwei unterschiedlichen Schichten in ihrem Gehalt an $La_2O_3$ unterscheiden, zur Herstellung dentaler Restaurationen.

2. Verwendung nach Anspruch 1, bei der mindestens eine Schicht 0,01 bis 1,0 Gew.-%, bevorzugt 0,025 bis 0,5 Gew.-% und am meisten bevorzugt 0,03 bis 0,20 Gew.-% $La_2O_3$ enthält.

3. Verwendung nach Anspruch 1 oder 2, bei der mindestens eine Schicht $Al_2O_3$ und/oder MgO enthält.

4. Verwendung nach Anspruch 3, bei der mindestens eine Schicht $La_2O_3$ enthält und mindestens eine andere Schicht $Al_2O_3$ und/oder MgO enthält.

5. Verwendung nach Anspruch 3 oder 4, bei der die mindestens eine Schicht 0,001 bis 5 Gew.-%, insbesondere 0,005 bis 1,0 Gew.-%, bevorzugt 0,01 bis 0,20 Gew.-% und am meisten bevorzugt 0,02 bis 0,10 Gew.-% $Al_2O_3$ und/oder MgO enthält.

**6.** Verwendung nach einem der Ansprüche 3 bis 5, bei der die mindestens eine Schicht 0,001 bis 5 Gew.-%, insbesondere 0,005 bis 1,0 Gew.-%, bevorzugt 0,01 bis 0,10 Gew.-% und am meisten bevorzugt 0,02 bis 0,05 Gew.-% $Al_2O_3$ enthält.

**7.** Verwendung nach einem der Ansprüche 3 bis 6, bei der die mindestens eine Schicht 0,001 bis 5 Gew.-%, insbesondere 0,005 bis 1,0 Gew.-%, bevorzugt 0,01 bis 0,10 Gew.-% und am meisten bevorzugt 0,01 bis 0,03 Gew.-% MgO enthält.

**8.** Verwendung nach einem der Ansprüche 3 bis 7, bei der die mindestens eine Schicht $Al_2O_3$ und MgO in einem Gewichtsverhältnis von 10:1 bis 1:10, vorzugsweise 5:1 bis 1:1 und besonders bevorzugt 4:1 bis 2:1 enthält.

**9.** Verwendung nach einem der Ansprüche 1 bis 8, bei der mindestens eine Schicht und vorzugsweise alle Schichten $Y_2O_3$ enthalten und insbesondere eine und vorzugsweise alle Schichten 0,1 bis 20,0 Gew.-%, insbesondere 1,0 bis 15,0 Gew.-%, bevorzugt 5,0 bis 12,5 Gew.-% und am meisten bevorzugt 7,0 bis 9,5 Gew.-% $Y_2O_3$ enthalten.

**10.** Verwendung nach Anspruch 9, bei der sich die mindestens zwei unterschiedlichen Schichten in ihrem Gehalt an $Y_2O_3$ unterscheiden, wobei vorzugsweise die Differenz im $Y_2O_3$-Gehalt zwischen der Schicht mit dem niedrigsten $Y_2O_3$-Gehalt und der Schicht mit dem höchsten $Y_2O_3$-Gehalt mindestens 1,0 Gew.-%, vorzugsweise mindestens 1,5 Gew.-% und insbesondere mindestens 1,8 Gew.-%, weiter bevorzugt mindestens 2,0 Gew.-% und insbesondere mindestens 2,5 Gew.-%, noch weiter bevorzugt mindestens 3,0 Gew.-% und insbesondere mindestens 3,5 Gew.-% und am meisten bevorzugt mindestens 3,8 Gew.-% beträgt.

**11.** Verwendung nach Anspruch 10, bei der die Schicht mit dem niedrigsten $Y_2O_3$-Gehalt 0,005 bis 1,0 Gew.-%, insbesondere 0,01 bis 1,0 Gew.-%, bevorzugt 0,25 bis 0,9 Gew.-% und am meisten bevorzugt 0,5 bis 0,8 Gew.-% $La_2O_3$ enthält.

**12.** Verwendung nach Anspruch 10 oder 11, bei der die Schicht mit dem höchsten $Y_2O_3$-Gehalt 0,001 bis 5 Gew.-%, insbesondere 0,005 bis 1,0 Gew.-%, bevorzugt 0,01 bis 0,20 Gew.-% und am meisten bevorzugt 0,02 bis 0,10 Gew.-% $Al_2O_3$ und/oder MgO enthält.

**13.** Verwendung nach einem der Ansprüche 9 bis 12, bei der sich in jeder der mindestens zwei unterschiedlichen Schichten der $La_2O_3$-Gewichtsanteil nach der folgenden Formel berechnet:

$$\mathtt{m(La_2O_3)\ =\ m_{min}(La_2O_3)\ +\ (m_{max}(Y_2O_3)-m(Y_2O_3))\ *\ f,}$$

wobei

$m(La_2O_3)$ der $La_2O_3$-Gewichtsanteil in der jeweiligen Schicht ist,
$m_{min}(La_2O_3)$ der minimale $La_2O_3$-Gewichtsanteil aller Schichten ist,
$m(Y_2O_3)$ der $Y_2O_3$-Gewichtsanteil in der jeweiligen Schicht ist,
$m_{max}(Y_2O_3)$ der maximale $Y_2O_3$-Gewichtsanteil aller Schichten ist, und
$f$ im Bereich von 0,01 bis 1,00, insbesondere im Bereich von 0,03 bis 0,20, bevorzugt im Bereich von 0,06 bis 0,10, besonders bevorzugt im Bereich von 0,065 bis 0,085 und am meisten bevorzugt im Bereich von 0,081 bis 0,083 liegt.

**14.** Verwendung nach einem der Ansprüche 1 bis 13, bei der der Rohling mindestens zwei unterschiedliche Schichten umfasst, die aus mindestens einem ersten Oxidkeramikmaterial und einem zweiten Oxidkeramikmaterial gebildet sind, wobei die Schichten der Oxidkeramikmaterialien eine kontinuierliche Änderung der Zusammensetzung von der Zusammensetzung des ersten Oxidkeramikmaterials zur Zusammensetzung des zweiten Oxidkeramikmaterials aufweisen.

**15.** Verwendung nach einem der Ansprüche 1 bis 14, bei der die Oxidkeramik auf stabilisiertem tetragonalem Zirkonoxid-Polykristall basiert.

**16.** Verwendung nach einem der Ansprüche 1 bis 15 zur Herstellung einer mehrgliedrigen Dentalrestauration, vorzugsweise einer Dentalrestauration, die zwei oder mehr Glieder umfasst, und insbesondere einer Brücke, die zwei oder mehr Glieder umfasst.

**17.** Verwendung nach einem der Ansprüche 1 bis 16, bei der die mindestens zwei unterschiedlichen Schichten unterschiedliche Farben haben.

**18.** Verwendung nach einem der Ansprüche 1 bis 17, bei der der Rohling einen Distorsionskoeffizienten

$$d = \frac{(\mathrm{HV}_{max} - \mathrm{HV}_{min})}{\overline{HV}}$$

von weniger als 0,4, insbesondere weniger als 0,35, insbesondere weniger als 0,3, vorzugsweise weniger 0,25, weiter bevorzugt weniger als 0,2 und am meisten bevorzugt weniger als 0,1 aufweist, wobei der Koeffizient auf Basis von mindestens einer Messung von HV für jede der unterschiedlichen Schichten berechnet wird, wobei:

HV die Vickers-Härte gemessen bei einer Kraft im Bereich von 2,5 bis 5,0 kgf (24,517 bis 49,034 N) und insbesondere bei einer Kraft von 5,0 kgf (49,034 N) gemäß ISO 14705:2008 ist;
$\mathrm{HV}_{max}$ das Maximum der gemessenen Werte von HV ist;
$\mathrm{HV}_{min}$ das Minimum der gemessenen Werte von HV ist; und
$\overline{HV}$ das arithmetische Mittel der gemessenen Werte von HV ist.

**19.** Verwendung eines mehrschichtigen Oxidkeramik-Grünkörpers, der mindestens zwei unterschiedliche Schichten umfasst, wobei die Oxidkeramik auf Zirkonoxid basiert und wobei mindestens eine Schicht 0,005 bis 1,0 Gew.-% $La_2O_3$ enthält und sich die mindestens zwei unterschiedlichen Schichten in ihrem Gehalt an $La_2O_3$ unterscheiden, zur Herstellung dentaler Restaurationen.

**20.** Verwendung nach Anspruch 19, bei der mindestens eine Schicht $Al_2O_3$ und/oder MgO enthält und vorzugsweise mindestens eine Schicht $La_2O_3$ enthält und mindestens eine andere Schicht $Al_2O_3$ und/oder MgO enthält.

**21.** Verwendung nach einem der Ansprüche 1 bis 20, bei der das Sinterverhalten der mindestens zwei unterschiedlichen Schichten so angeglichen ist, dass der Rohling oder Grünkörper verzugsfrei sintern kann.

**22.** Verfahren zur Herstellung eines wie in einem der Ansprüche 1 bis 18 oder 21 definierten Rohlings oder eines wie in einem der Ansprüche 19 bis 21 definierten Grünkörpers, bei dem

(a) mindestens ein erstes Oxidkeramikmaterial und ein zweites Oxidkeramikmaterial bereitgestellt werden, die sich in ihrer chemischen Zusammensetzung unterscheiden,
(b) mindestens einem der Oxidkeramikmaterialien $La_2O_3$ zugefügt wird und
(c) gegebenenfalls mindestens einem der Oxidkeramikmaterialien $Al_2O_3$ und/oder MgO zugefügt wird.

**23.** Verfahren nach Anspruch 22, bei dem mindestens ein Oxidkeramikmaterial mit $La_2O_3$ infiltriert oder beschichtet wird und/oder mindestens ein Oxidkeramikmaterial mit $Al_2O_3$ und/oder MgO infiltriert oder beschichtet wird.

**24.** Verfahren nach einem der Ansprüche 22 oder 23, bei dem ferner

(d) Schichten der Oxidkeramikmaterialien gebildet und die Schichten aufeinander angeordnet werden,
(e) die Oxidkeramikmaterialien kompaktiert und insbesondere verpresst werden, um den Grünkörper zu erhalten, und
(f) gegebenenfalls der Grünkörper vorgesintert wird, um den vorgesinterten Keramikrohling zu erhalten.

**25.** Verfahren nach Anspruch 24, bei dem die Schichten der Oxidkeramikmaterialien eine kontinuierliche Änderung der Zusammensetzung von der Zusammensetzung des ersten Oxidkeramikmaterials zur Zusammensetzung des zweiten Oxidkeramikmaterials aufweisen.

**26.** Verwendung nach einem der Ansprüche 1 bis 21, wobei der Rohling oder Grünkörper gemäß einem Verfahren nach einem der Ansprüche 22 bis 25 erhältlich ist.

**27.** Verfahren zur Herstellung einer Dentalrestauration, bei dem ein wie in einem der Ansprüche 1 bis 18, 21 oder 26 definierter Rohling oder ein wie in einem der Ansprüche 19 bis 21 oder 26 definierter Grünkörper verwendet wird.

**28.** Verfahren nach Anspruch 27, bei dem dem Rohling oder dem Grünkörper die Form einer gewünschten Geometrie gegeben wird, um ein geformtes Keramikprodukt zu erhalten, wobei die Formgebung vorzugsweise durch maschinelle Bearbeitung und besonders bevorzugt unter Verwendung eines CAD/CAM-Verfahrens durchgeführt wird.

**29.** Verfahren nach Anspruch 28, bei dem das geformte Keramikprodukt die Form eines dentalen Gerüsts oder Stiftaufbaus oder einer monolithischen vollanatomischen Dentalrestauration, insbesondere einer mehrgliedrigen Dentalrestauration, aufweist.

**30.** Verfahren nach Anspruch 28 oder 29, bei dem ferner das geformte Keramikprodukt dichtgesintert wird.


**Claims**

**1.** Use of a presintered multilayer oxide ceramic blank for the production of dental restorations, which comprises at least two different layers, wherein the oxide ceramic is based on zirconia and at least one layer comprises 0.005 to 5 wt.-% $La_2O_3$ and the at least two different layers differ in their content of $La_2O_3$.

**2.** Use according to claim 1, wherein at least one layer comprises 0.01 to 1.0 wt.-%, preferably 0.025 to 0.5 wt.-% and most preferably 0.03 to 0.20 wt.-% $La_2O_3$.

**3.** Use according to claim 1 or 2, wherein at least one layer comprises $Al_2O_3$ and/or MgO.

**4.** Use according to claim 3, wherein at least one layer comprises $La_2O_3$ and at least one other layer comprises $Al_2O_3$ and/or MgO.

**5.** Use according to claim 3 or 4, wherein the at least one layer comprises 0.001 to 5 wt.-%, in particular 0.005 to 1.0 wt.-%, preferably 0.01 to 0.20 wt.-% and most preferably 0.02 to 0.10 wt.-% $Al_2O_3$ and/or MgO.

**6.** Use according to any one of claim 3 to 5, wherein the at least one layer comprises 0.001 to 5 wt.-%, in particular 0.005 to 1.0 wt.-%, preferably 0.01 to 0.10 wt.-% and most preferably 0.02 to 0.05 wt.-% $Al_2O_3$.

**7.** Use according to any one of claim 3 to 6, wherein the at least one layer comprises 0.001 to 5 wt.-%, in particular 0.005 to 1.0 wt.-%, preferably 0.01 to 0.10 wt.-% and most preferably 0.01 to 0.03 wt.-% MgO.

**8.** Use according to any one of claim 3 to 7, wherein the at least one layer comprises $Al_2O_3$ and MgO in a weight ratio of 10:1 to 1:10, preferably 5:1 to 1:1 and particularly preferably 4:1 to 2:1.

**9.** Use according to any one of claims 1 to 8, wherein at least one layer and preferably all layers comprise $Y_2O_3$ and in particular one and preferably all layers comprise 0.1 to 20.0 wt.-%, in particular 1.0 to 15.0 wt.-%, preferably 5.0 to 12.5 wt.-% and most preferably 7.0 to 9.5 wt.-% $Y_2O_3$.

**10.** Use according to claim 9, wherein the at least two different layers differ in their content of $Y_2O_3$, wherein preferably the difference in the $Y_2O_3$ content between the layer with the lowest $Y_2O_3$ content and the layer with the highest $Y_2O_3$ content is at least 1.0 wt.-%, preferably at least 1.5 wt.-% and in particular at least 1.8 wt.-%, further preferred at least 2.0 wt.-% and in particular at least 2.5 wt.-%, even more preferred at least 3.0 wt.-% and in particular at least 3.5 wt.-% and most preferred at least 3.8 wt.-%.

**11.** Use according to claim 10, wherein the layer with the lowest $Y_2O_3$ content comprises 0.005 to 1.0 wt.-%, in particular 0.01 to 1.0 wt.-%, preferably 0.25 to 0.9 wt.-% and most preferably 0.5 to 0.8 wt.-% $La_2O_3$.

**12.** Use according to claim 10 or 11, wherein the layer with the highest $Y_2O_3$ content comprises 0.001 to 5 wt.-%, in particular 0.005 to 1.0 wt.-%, preferably 0.01 to 0.20 wt.-% and most preferably 0.02 to 0.1 wt.-% $Al_2O_3$ and/or MgO.

**13.** Use according to any one of claims 9 to 12, wherein the proportion by weight of $La_2O_3$ in each of the at least two different layers is calculated according to the following formula:

$$m(La_2O_3) = m_{min}(La_2O_3) + (m_{max}(Y_2O_3) - m(Y_2O_3)) * f,$$

wherein

$m(La_2O_3)$ is the proportion by weight of $La_2O_3$ in the respective layer,
$m_{min}(La_2O_3)$ is the minimum proportion by weight of $La_2O_3$ of all layers,
$m(Y_2O_3)$ is the proportion by weight of $Y_2O_3$ in the respective layer,
$m_{max}(Y_2O_3)$ is the maximum proportion by weight of $Y_2O_3$ of all layers, and
f is in the range of from 0.01 to 1.00, in particular in the range of from 0.03 to 0.20, preferably in the range of from 0.06 to 0.10, particularly preferably in the range of from 0.065 to 0.085 and most preferably in the range of from 0.081 to 0.083.

14. Use according to any one of claims 1 to 13, in which the blank comprises at least two different layers formed by at least one oxide ceramic material and at least one second oxide ceramic material, wherein the layers of the oxide ceramic materials exhibit a continuous change of composition from the composition of the first oxide ceramic material to the composition of the second oxide ceramic material.

15. Use according to any one of claims 1 to 14, wherein the oxide ceramic is based on stabilized tetragonal zirconia polycrystal.

16. Use according to any one of claims 1 to 15 for the production of a multi-unit dental restoration, preferably a dental restoration which comprises two or more units, and in particular a bridge which comprises two or more units.

17. Use according to any one of claims 1 to 16, wherein the at least two different layers have different colours.

18. Use according to any one of claims 1 to 17, wherein the blank has a coefficient of distortion

$$d = \frac{(HV_{max} - HV_{min})}{\overline{HV}}$$

of less than 0.4, in particular less than 0.35, in particular less than 0.3, preferably less than 0.25, further preferably less than 0.2 and most preferably less than 0.1, wherein the coefficient is calculated on the basis of at least one measurement of HV for each of the different layers,
wherein:

HV is the Vickers hardness measured at a load in the range of from 2.5 to 5.0 kgf (24.517 to 49.034 N) and in particular at a load of 5.0 kgf (49.034 N) in accordance with ISO 14705:2008;
$HV_{max}$ is the maximum of the measured values of HV;
$HV_{min}$ is the minimum of the measured values of HV; and
$\overline{HV}$ is the arithmetic mean of the measured values of HV.

19. Use of a multilayer oxide ceramic green body for the production of dental restorations, which comprises at least two different layers, wherein the oxide ceramic is based on zirconia and at least one layer comprises 0.005 to 1.0 wt.-% $La_2O_3$ and the at least two different layers differ in their content of $La_2O_3$.

20. Use according to claim 19, wherein at least one layer comprises $Al_2O_3$ and/or MgO and preferably at least one layer comprises $La_2O_3$ and at least one other layer comprises $Al_2O_3$ and/or MgO.

21. Use according to any one of claims 1 to 20, wherein the sintering behaviour of the at least two different layers is aligned such that the blank or green body can sinter without distortion.

22. Process for the production of a blank as defined in any one of claims 1 to 18 or 21 or of a green body as defined in any one of claims 19 to 21, in which

(a) at least one first oxide ceramic material and one second oxide ceramic material are provided which differ in terms of their chemical composition,
(b) $La_2O_3$ is added to at least one of the oxide ceramic materials, and
(c) optionally, $Al_2O_3$ and/or MgO is added to at least one oxide ceramic material.

**23.** Process according to claim 22, in which at least one oxide ceramic material is infiltrated or coated with $La_2O_3$ and/or at least one oxide ceramic material is infiltrated or coated with $Al_2O_3$ and/or MgO.

**24.** Process according to any one of claims 22 or 23, in which furthermore

(d) layers of the oxide ceramic materials are formed and the layers are arranged one on the other,
(e) the oxide ceramic materials are compacted and in particular compressed in order to obtain the green body, and
(f) optionally, the green body is presintered in order to obtain the presintered ceramic blank.

**25.** Process according to claim 24, in which the layers of the oxide ceramic materials exhibit a continuous change of composition from the composition of the first oxide ceramic material to the composition of the second oxide ceramic material.

**26.** Use according to any one of claims 1 to 21, wherein the blank or green body is obtainable by a process according to any one of claims 22 to 25.

**27.** Process for the production of a dental restoration, in which a blank as defined in any one of claims 1 to 18, 21 or 26 or a green body as defined in any one of claims 19 to 21 or 26 is used.

**28.** Process according to claim 27, in which the blank or the green body is given the shape of a desired geometry in order to obtain a shaped ceramic product, wherein the shaping is preferably carried out by machining and particularly preferably using a CAD/CAM process.

**29.** Process according to claim 28, in which the shaped ceramic product has the shape of a dental framework or abutment or of a monolithic fully anatomical dental restoration, in particular a multi-unit dental restoration.

**30.** Process according to claim 28 or 29, in which furthermore the shaped ceramic product is densely sintered.

**Revendications**

**1.** Utilisation d'une ébauche pré-frittée multicouche en céramique oxyde, qui comprend au moins deux couches différentes et dans laquelle la céramique oxyde est à base d'oxyde de zirconium, au moins une couche contient de 0,005 à 1,0 % en poids de $La_2O_3$ et les couches différentes, au nombre d'au moins deux, se distinguent par leur teneur en $La_2O_3$, pour la fabrication de pièces de restauration dentaire.

**2.** Utilisation conforme à la revendication 1, dans laquelle au moins une couche contient de 0,01 à 1,0 % en poids, de préférence de 0,025 à 0,5 % en poids, et au mieux de 0,03 à 0,20 % en poids de $La_2O_3$.

**3.** Utilisation conforme à la revendication 1 ou 2, dans laquelle au moins une couche contient de l'$Al_2O_3$ et/ou de la MgO.

**4.** Utilisation conforme à la revendication 3, dans laquelle au moins une couche contient du $La_2O_3$ et au moins une autre couche contient de l'$Al_2O_3$ et/ou de la MgO.

**5.** Utilisation conforme à la revendication 3 ou 4, dans laquelle la couche au nombre d'au moins une contient de 0,001 à 5 % en poids, en particulier de 0,005 à 1,0 % en poids, de préférence de 0,01 à 0,20 % en poids, et au mieux de 0,02 à 0,10 % en poids d'$Al_2O_3$ et/ou de MgO.

**6.** Utilisation conforme à l'une des revendications 3 à 5, dans laquelle la couche au nombre d'au moins une contient de 0,001 à 5 % en poids, en particulier de 0,005 à 1,0 % en poids, de préférence de 0,01 à 0,10 % en poids, et au mieux de 0,02 à 0,05 % en poids d'$Al_2O_3$.

**7.** Utilisation conforme à l'une des revendications 3 à 6, dans laquelle la couche au nombre d'au moins une contient de 0,001 à 5 % en poids, en particulier de 0,005 à 1,0 % en poids, de préférence de 0,01 à 0,10 % en poids, et au mieux de 0,01 à 0,03 % en poids de MgO.

**8.** Utilisation conforme à l'une des revendications 3 à 7, dans laquelle la couche au nombre d'au moins une contient de l'$Al_2O_3$ et de la MgO en un rapport pondéral de 10/1 à 1/10, de préférence de 5/1 à 1/1, et mieux encore de 4/1 à 2/1.

**9.** Utilisation conforme à l'une des revendications 1 à 8, dans laquelle au moins une couche, et de préférence toutes les couches, contient ou contiennent de l'$Y_2O_3$, et en particulier, dans laquelle une couche, et de préférence toutes les couches, contient ou contiennent de 0,1 à 20,0 % en poids, en particulier de 1,0 à 15,0 % en poids, de préférence de 5,0 à 12,5 % en poids, et au mieux de 7,0 à 9,5 % en poids d'$Y_2O_3$.

**10.** Utilisation conforme à la revendication 9, dans laquelle les différentes couches, au nombre d'au moins deux, se distinguent par leur teneur en $Y_2O_3$, étant entendu qu'il est avantageux que la différence des teneurs en $Y_2O_3$ entre la couche présentant la plus faible teneur en $Y_2O_3$ et la couche présentant la plus forte teneur en $Y_2O_3$ vaille au moins 1,0 % en poids, de préférence au moins 1,5 % en poids et en particulier au moins 1,8 % en poids, mieux encore au moins 2,0 % en poids et en particulier au moins 2,5 % en poids, encore mieux au moins 3,0 % en poids et en particulier au moins 3,5 % en poids, et au mieux au moins 3,8 % en poids.

**11.** Utilisation conforme à la revendication 10, dans laquelle la couche présentant la plus faible teneur en $Y_2O_3$ contient de 0,005 à 1,0 % en poids, en particulier de 0,01 à 1,0 % en poids, de préférence de 0,25 à 0,9 % en poids, et au mieux de 0,5 à 0,8 % en poids de $La_2O_3$.

**12.** Utilisation conforme à la revendication 10 ou 11, dans laquelle la couche présentant la plus forte teneur en $Y_2O_3$ contient de 0,001 à 5 % en poids, en particulier de 0,005 à 1,0 % en poids, de préférence de 0,01 à 0,20 % en poids, et au mieux de 0,02 à 0,10 % en poids d'$Al_2O_3$ et/ou de MgO.

**13.** Utilisation conforme à l'une des revendications 9 à 12, dans laquelle, dans chacune des différentes couches au nombre d'au moins deux, la proportion pondérale de $La_2O_3$ est calculée selon la formule suivante :

$$m(La_2O_3) = m_{min}(La_2O_3) + (m_{max}(Y_2O_3) - m(Y_2O_3)).\mathbf{f}$$

dans laquelle

- $m(La_2O_3)$ représente la proportion pondérale de $La_2O_3$ dans la couche considérée,
- $m_{min}(La_2O_3)$ représente la valeur minimale de la proportion pondérale de $La_2O_3$ dans toutes les couches,
- $m(Y_2O_3)$ représente la proportion pondérale d'$Y_2O_3$ dans la couche considérée,
- $m_{max}(Y_2O_3)$ représente la valeur maximale de la proportion pondérale d'$Y_2O_3$ dans toutes les couches,
- et le facteur f se situe dans l'intervalle allant de 0,01 à 1,00, en particulier dans l'intervalle allant de 0,03 à 0,20, de préférence dans l'intervalle allant de 0,06 à 0,10, mieux encore dans l'intervalle allant de 0,065 à 0,085, et au mieux dans l'intervalle allant de 0,081 à 0,083.

**14.** Utilisation conforme à l'une des revendications 1 à 13, dans laquelle l'ébauche comprend au moins deux couches différentes qui sont constituées d'au moins un premier matériau céramique oxyde et un deuxième matériau céramique oxyde, et les couches de matériau céramique oxyde présentent une variation continue de composition, depuis la composition du premier matériau céramique oxyde jusqu'à la composition du deuxième matériau céramique oxyde.

**15.** Utilisation conforme à l'une des revendications 1 à 14, dans laquelle la céramique oxyde est à base d'oxyde de zirconium poly-cristallin tétragonal stabilisé.

**16.** Utilisation conforme à l'une des revendications 1 à 15, pour la fabrication d'une pièce de restauration dentaire multi-éléments, de préférence, d'une pièce de restauration dentaire qui comprend deux éléments ou plus, et en particulier, d'un bridge qui comprend deux éléments ou plus.

**17.** Utilisation conforme à l'une des revendications 1 à 16, dans laquelle les différentes couches, au nombre d'au moins deux, ont des couleurs différentes.

**18.** Utilisation conforme à l'une des revendications 1 à 17, dans laquelle l'ébauche présente un coefficient de distorsion

$$d = \frac{(HV_{max} - HV_{min})}{HV}$$

valant moins de 0,4, en particulier moins de 0,35, tout particulièrement moins de 0,3, de préférence moins de 0,25,

**EP 3 558 671 B1**

mieux encore moins de 0,2 et au mieux moins de 0,1, étant entendu que ce coefficient est calculé à partir d'au moins une mesure de HV pour chacune des différentes couches, et que :

- HV désigne la dureté Vickers, mesurée avec une force située dans l'intervalle allant de 2,5 à 5,0 kgf (de 24,517 à 49,034 N), en particulier avec une force valant 5,0 kgf (49,034 N), selon la norme ISO 14705:2008,
- $HV_{max}$ désigne la valeur maximale parmi les valeurs de HV mesurées,
- $HV_{min}$ désigne la valeur minimale parmi les valeurs de HV mesurées,
- et $\overline{HV}$ désigne la moyenne arithmétique des valeurs de HV mesurées.

19. Utilisation d'un corps cru multicouche en céramique oxyde, qui comprend au moins deux couches différentes et dans laquelle la céramique oxyde est à base d'oxyde de zirconium, au moins une couche contient de 0,005 à 1,0 % en poids de $La_2O_3$ et les couches différentes, au nombre d'au moins deux, se distinguent par leur teneur en $La_2O_3$, pour la fabrication de pièces de restauration dentaire.

20. Utilisation conforme à la revendication 19, dans laquelle au moins une couche contient de l'$Al_2O_3$ et/ou de la MgO, et de préférence, au moins une couche contient du $La_2O_3$ et au moins une autre couche contient de l'$Al_2O_3$ et/ou de la MgO.

21. Utilisation conforme à l'une des revendications 1 à 20, dans laquelle la tenue au frittage des différentes couches, au nombre d'au moins deux, est ajustée de telle sorte que l'ébauche ou le corps cru peut être fritté(e) sans se déformer.

22. Procédé de fabrication d'une ébauche, telle que définie dans l'une des revendications 1 à 18 et 21, ou d'un corps cru, tel que défini dans l'une des revendications 19 à 21, dans lequel

   a) on prend au moins un premier matériau céramique oxyde et un deuxième matériau céramique oxyde qui se distinguent par leur composition chimique,
   b) on ajoute du $La_2O_3$ à au moins l'un de ces matériaux oxydes céramiques,
   c) et en option, on ajoute de l'$Al_2O_3$ et/ou de la MgO à au moins l'un de ces matériaux oxydes céramiques.

23. Procédé conforme à la revendication 22, dans lequel au moins un matériau oxyde céramique est infiltré ou enrobé de $La_2O_3$, et/ou au moins un matériau oxyde céramique est infiltré ou enrobé d'$Al_2O_3$ et/ou de MgO.

24. Procédé conforme à l'une des revendications 22 ou 23, dans lequel, en outre,

   d) on forme des couches des matériaux oxydes céramiques et l'on dispose ces couches les unes au-dessus des autres,
   e) on compacte les matériaux oxydes céramiques, et en particulier on les comprime, afin d'obtenir un corps cru,
   f) et en option, on soumet ce corps cru à un pré-frittage, afin d'obtenir une ébauche en céramique pré-frittée.

25. Procédé conforme à la revendication 24, dans lequel les couches de matériau oxyde céramique présentent une variation continue de composition, depuis la composition du premier matériau céramique oxyde jusqu'à la composition du deuxième matériau céramique oxyde.

26. Utilisation conforme à l'une des revendications 1 à 21, dans laquelle l'ébauche ou le corps cru est accessible selon un procédé conforme à l'une des revendications 22 à 25.

27. Procédé de fabrication d'une pièce de restauration dentaire, dans lequel on utilise une ébauche telle que définie dans l'une des revendications 1 à 18, 21 et 26, ou un corps cru tel que défini dans l'une des revendications 19 à 21 et 26.

28. Procédé conforme à la revendication 27, dans lequel on donne à l'ébauche ou au corps cru la forme géométrique voulue pour obtenir un produit céramique façonné, étant entendu que l'on réalise de préférence cette mise en forme en travaillant sur machine, et de manière particulièrement préférée, en utilisant un procédé CAD/CAM (conception et fabrication assistées par ordinateur).

29. Procédé conforme à la revendication 28, dans lequel le produit céramique façonné présente la forme d'une armature ou structure pivot dentaire ou d'une pièce de restauration dentaire monolithique entièrement anatomique, en particulier d'une pièce de restauration dentaire multi-éléments.

**30.** Procédé conforme à la revendication 28 ou 29, dans lequel le produit céramique façonné est en outre densément fritté.

Fig. 1

A

B

0.03 mm

1 mm

Fig. 2

A

B

0,00 mm

1 mm

Fig. 3

A

B

0,36 mm

1 mm

Fig. 4

A

B

0,04 mm

1 mm

Fig. 5

A

B

Fig. 6

A

B

0,00 mm

1 mm

Fig. 7

Fig. 8

Fig. 9

A

B

0,00 mm

1mm

Fig. 10

A

B

0,00 mm

Fig. 11

A

B

Fig. 12

A

B

0,00 mm

1 mm

Fig. 13

A

B

Fig. 14

A

2 mm

B

0,0 mm

1 mm

Fig. 15

Fig. 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6709694 B1
- EP 1486476 A1
- US 5011403 A
- US 5263858 A
- US 5656564 A
- US 6713421 A
- US 2007292597 A1
- US 20080274440 A1
- US 6379593 B
- US 2007272120 A1
- US 2008064011 A1
- WO 2008083358 A1
- US 20100216095 A1
- US 20110189636 A1
- US 2012139141 A1
- WO 2015011079 A1
- US 2015282905 A
- US 2014135200 A1

**In der Beschreibung aufgeführte Nicht-Patentliteratur**

- **HANNINK et al.** *J. Am. Ceram. Soc.,* 2000, vol. 83, 461-487
- **DENRY et al.** *Dental Materials,* 2008, vol. 24, 299-307